(19) Europäisches Patentamt
European Patent Office
Office européen des brevets

(11) EP 2 459 518 B1

(12) # FASCICULE DE BREVET EUROPEEN

(45) Date de publication et mention de la délivrance du brevet:
**11.12.2013 Bulletin 2013/50**

(21) Numéro de dépôt: **10734192.7**

(22) Date de dépôt: **06.07.2010**

(51) Int Cl.:
***C07C 213/10** (2006.01)* ***C07C 215/08** (2006.01)*
***B01D 53/14** (2006.01)* ***B01D 53/62** (2006.01)*
***B01D 53/78** (2006.01)* ***C07D 249/04** (2006.01)*
***C07D 249/12** (2006.01)* ***C07D 257/04** (2006.01)*

(86) Numéro de dépôt international:
**PCT/FR2010/000492**

(87) Numéro de publication internationale:
**WO 2011/012777 (03.02.2011 Gazette 2011/05)**

(54) **SOLUTION ABSORBANTE CONTENANT UN INHIBITEUR DE DEGRADATION DERIVE D'UN TRIAZOLE OU D'UN TETRAZOLE ET PROCEDE D'ABSORPTION DE COMPOSES ACIDES CONTENUS DANS UN EFFLUENT GAZEUX**

ABSORPTIONSLÖSUNG MIT EINEM ABBAUHEMMER AUS EINEM TRIAZOL ODER TETRAZOL UND VERFAHREN ZUR ABSORPTION VON SÄUREVERBINDUNGEN IN EINEM ABGAS

ABSORBENT SOLUTION CONTAINING A DEGRADATION INHIBITOR DERIVED FROM A TRIAZOLE OR FROM A TETRAZOLE AND PROCESS FOR THE ABSORPTION OF ACID COMPOUNDS CONTAINED IN A GASEOUS EFFLUENT

(84) Etats contractants désignés:
**AL AT BE BG CH CY CZ DE DK EE ES FI FR GB GR HR HU IE IS IT LI LT LU LV MC MK MT NL NO PL PT RO SE SI SK SM TR**

(30) Priorité: **31.07.2009 FR 0903812**

(43) Date de publication de la demande:
**06.06.2012 Bulletin 2012/23**

(73) Titulaire: **IFP Energies nouvelles**
**92852 Rueil-Malmaison Cedex (FR)**

(72) Inventeurs:
- **CARRETTE, Pierre-Louis**
  **F-69005 Lyon (FR)**
- **DELFORT, Bruno**
  **F-75005 Paris (FR)**

(56) Documents cités:
**EP-A- 1 582 250** **FR-A- 2 909 010**

EP 2 459 518 B1

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

Printed by Jouve, 75001 PARIS (FR)

## Description

La présente invention concerne le domaine de la désacidification d'un effluent gazeux. Plus précisément la présente invention propose des composés pour réduire la dégradation d'une solution absorbante mise en oeuvre pour absorber les composés acides contenus dans un effluent gazeux, la solution absorbante comportant des amines en solution aqueuse. En particulier, l'invention concerne des composés utilisés pour réduire la dégradation des amines utilisées pour la désacidification de gaz contenant de l'oxygène, comme par exemple les fumées de combustion.

La désacidification des effluents gazeux, tels que par exemple le gaz naturel et les fumées de combustion, est généralement réalisée par lavage par une solution absorbante. La solution absorbante permet d'absorber les composés acides présents dans l'effluent gazeux ($H_2S$, mercaptans, $CO_2$, COS, $SO_2$, $CS_2$).

La désacidifcation de ces effluents, notamment la décarbonatation et la désulfuration, impose des contraintes spécifiques à la solution absorbante, en particulier une stabitité thermique et chimique notamment face aux impuretés de l'effluent, à savoir essentiellement l'oxygène, les SOx et les NOx. L'oxygène peut aussi entrer en contact avec la solution absorbante sans être forcément présent dans l'effluent gazeux à traiter comme dans le cas par exemple d'une entrée accidentelle d'air au niveau des bacs de stockage de solution absorbante.

Les solutions absorbantes les plus utilisées aujourd'hui sont les solutions aqueuses d'alcanolamines. On peut citer le document FR 2 820 430 qui propose des procédés de désacidification d'effluents gazeux.

Toutefois, il est bien connu de l'homme de l'art que ces amines présentent l'inconvénient de se dégrader dans les conditions de mise en oeuvre.

En particulier, les amines peuvent être dégradées par l'oxygène engendrant une consommation de l'amine et la formation de produits de dégradation qui s'accumulent dans l'unité ou, pour les plus volatils, qui sont entraînés dans les effluents gazeux du procédé. Ainsi, en particulier dans le cas du traitement de fumées en post-combustion dans un procédé utilisant une solution aqueuse de monoéthanolamine (MEA) des quantités importantes d'ammoniac sont formées. L'ammoniac ainsi formé est entraîné dans l'atmosphère avec les fumées traitées ce qui pose des problèmes quant à la protection de l'environnement.

Dans le cas du captage du $CO_2$ dans les fumées issues d'unités industrielles ou de production d'électricité ou d'énergie en général, les phénomènes de dégradation de la solution absorbante aux amines sont accrus par la présence d'une quantité massive d'oxygène dans la charge à traiter pouvant aller jusqu'à 5% en volume en général. Dans le cas de fumées issues de cycle combiné au gaz naturel, la teneur volumique d'oxygène dans les fumées peut atteindre 15%.

La solution dégradée se caractérise par :

- une baisse de l'absorption des composés acides de la charge par rapport à une solution fraîche d'amine,
- une augmentation de la densité de la solution absorbante, ainsi que de sa viscosité, pouvant entraîner une perte de performance,
- la formation d'amines plus volatiles polluant le gaz traité et le gaz acide issu de l'étape de régénération : ammoniac, méthylamine, diméthylamine et triméthylamine par exemple selon la nature de l'amine utilisée,
- une accumulation de produits de dégradation dans la solution absorbante qui peut entraîner la nécessité d'un traitement de la solution dégradée,
- d'éventuels problèmes de moussage dus aux produits de dégradation. La dégradation de la solution absorbante pénalise donc les performances et le bon fonctionnement des unités de désacidification des gaz.

Pour pallier le problème de dégradation, à défaut de pouvoir limiter ou supprimer la présence d'oxygène dans la solution absorbante, on ajoute, dans la solution absorbante, des composés dont le rôle est de prévenir ou limiter les phénomènes de dégradation des composés amines, notamment la dégradation engendrée par les phénomènes d'oxydation. Ces composés sont couramment nommés agents ou additifs inhibiteurs de dégradation. Les principaux modes d'action connus des agent inhibiteurs de dégradation consistent selon leur nature en une réaction de type réduction et/ou en un captage, un piégeage et/ou une stabilisation des radicaux formés dans la solution absorbante afin de limiter ou d'empêcher ou d'interrompre les réactions, notamment les réactions en chaîne, de dégradation.

Le brevets US 5686016 cite des additifs utilisés pour limiter la dégradation de solutions absorbantes utilisées pour la désacidification du gaz naturel, en particulier les oximes.

Le brevet US 7056482 cite des additifs utilisés pour limiter la dégradation de solutions absorbantes utilisées pour le captage du $CO_2$, en particulier les thiosulfates et les sulfites.

Le brevet européen EP 1 582 250 cite des additifs du type triazoles hydroxylés pour limiter la dégradation de solutions absorbantes pour la desacidification de fluides.

De manière générale, la présente invention propose une famille d'agents inhibiteurs de dégradation qui permet notamment de réduire la dégradation d'une solution absorbante mise en oeuvre pour l'absorption de composés acides contenus dans un effluent gazeux, la solution absorbante comportant des composés amines en solution aqueuse.

La présente invention décrit une solution absorbante pour absorber les composés acides d'un effluent gazeux,

ladite solution comportant :

a) au moins une amine,
b) de l'eau,
c) au moins un composé inhibiteur de dégradation pour limiter la dégradation de ladite amine, le composé inhibiteur de dégradation étant un dérivé d'un triazole ou d'un tétrazole, dont au moins un substituant comporte un atome de soufre.

Selon l'invention, ledit composé inhibiteur de dégradation peut répondre à l'une des formules générales suivantes :

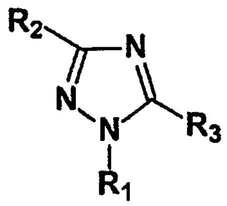
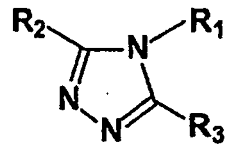
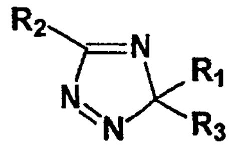

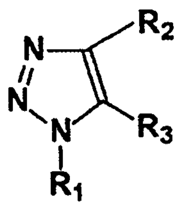
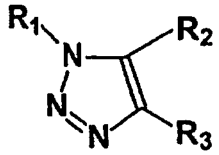
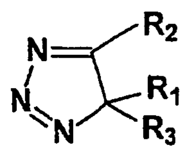

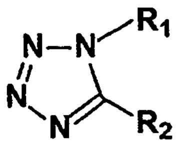
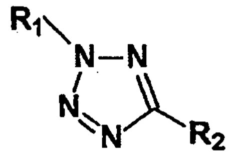
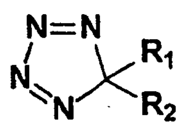

dans laquelle le radical R1 est choisi parmi :

○ un atome d'hydrogène,
○ un radical hydrocarboné comprenant 1 à 12 atomes de carbone,
○ un radical amino de formule générale -NR4R5 dans lequel le radical R4 et le radical R5 sont indépendamment choisis parmi :

  ○ un atome d'hydrogène,
  ○ un radical hydrocarboné comprenant 1 à 12 atomes de carbone,

et dans laquelle chacun des radicaux R2 et R3 est choisi indépendamment parmi les éléments suivants :

a) -S-X dans lequel le radical X est choisi parmi :

  ○ un atome d'hydrogène,
  ○ un élément alcalin ou alcalino-terreux,
  ○ un métal monovalent ou multivalent,
  ○ un cation ammonium NH4+ ou résultant de la protonation d'une fonction amine,
  ○ un cation phosphonium,
  ○ un radical hydrocarboné comprenant 1 à 12 atomes de carbone,
  ○ un radical choisi parmi un radical thiyl-triazole, thio-triazole, thiyl-tétrazole et thio-tétrazole,

b) un atome d'hydrogène,
c) un radical hydroxyle,

d) un radical amino de formule générale $-NR_4R_5$ dans lequel le radical $R_4$ et le radical $R_5$ sont indépendamment choisis parmi :

- ○ un atome d'hydrogène,
- ○ un radical hydrocarboné comprenant 1 à 12 atomes de carbone,

e) un radical comprenant 1 à 12 atomes de carbone.

Au moins l'un des radicaux $R_1$, $R_2$, $R_3$, $R_4$, $R_5$ et X peut être un groupe hydrocarboné renfermant entre 1 et 12 atomes de carbone et peut contenir, en outre, au moins un composé choisi parmi un hétéroatome et un halogène.

Au moins l'un des radicaux $R_1$, $R_2$, $R_3$, $R_4$, $R_5$ et X peut comporter au moins une fonction choisie parmi le groupe : une fonction hydroxyle, une fonction cétone, une fonction carboxylique, une fonction amine et une fonction nitrile.

Au moins deux radicaux choisis parmi $R_1$, $R_2$, $R_3$, $R_4$, $R_5$ et X peuvent être des groupements hydrocarbonés reliés par une liaison covalente pour former un cycle constitué de 5, 6, 7 ou 8 atomes.

La solution peut comporter entre 10% et 99% poids d'amine, entre 1% et 90% poids d'eau et entre 5 ppm et 5% poids de composé inhibiteur de dégradation.

Le composé inhibiteur de dégradation peut être choisi parmi le groupe contenant: le 1H-1,2,4-triazole-3-thiol, un sel du 1H-1,2,4-triazole-3-thiol, le 5-phényl-1H-1,2,4-triazole-3-thiol, un sel du 5-phényl-1H-1,2,4-triazole-3-thiol, le 5-(4-pyridyl)-1H-1,2,4-triazole-3-thiol, un sel du 5-(4-pyridyl)-1H-1,2,4-triazole-3-thiol, le 5-(3-pyridyl)-1H-1,2,4-triazole-3-thiol, un sel du 5-(3-pyridyl)-1H-1,2,4-triazole-3-thiol, le 4-méthyl-4H-1,2,4-triazole-3-thiol, un sel du 4-méthyl-4H-1,2,4-triazole-3-thiol, le 4-méthyl-5-(2-thienyl)-4H-1,2,4-triazole-3-thiol, un sel du 4-méthyl-5-(2-thienyl)-4H-1,2,4-triazole-3-thiol, le 4-méthyl-5-(3-thienylméthyl)-4H-1,2,4-triazole-3-thiol, un sel du 4-méthyl-5-(3-thienylméthyl)-4H-1,2,4-triazole-3-thiol, le 4-cyclohexyl-5-sulfanyl-4H-1,2,4-triazol-3-ol, un sel du 4-cyclohexyl-5-sulfanyl-4H-1,2,4-triazol-3-ol, le 3-amino-1,2,4-triazole-5-thiol, un sel du 3-amino-1,2,4-triazole-5-thiol, le 4-amino-4H-1,2,4-triazole-3,5-dithiol, un sel du 4-amino-4H-1,2,4-triazole-3,5-dithiol, le [1,2,4]triazolo[4,3-a]pyridine-3-thiol, un sel du [1,2,4]triazolo[4,3-a]pyridine-3-thiol, le 1H-5-mercapto-1,2,3-triazole, un sel du 1H-5-mercapto-1,2,3-triazole, le 1-méthyl-1H-tétrazole-5-thiol, un sel du 1-méthyl-1H-tétrazole-5-thiol, le 1-éthyl-1H-tétrazole-5-thiol, un sel du 1-éthyl-1H-tétrazole-5-thiol, le 1-phényl-1H-tétrazole-5-thiol, un sel du 1-phényl-1H-tétrazole-5-thiol, le 1-(4-hydroxyphényl)-1H-tétrazole-5-thiol, un sel du 1-(4-hydroxyphényl)-1H-tétrazole-5-thiol, l'acide 5-mercapto-1-tétrazoleacétique, un sel de l'acide 5-mercapto-1-tétrazoleacétique, le 1-[2-(diméthylamino)éthyl]-1H-tétrazole-5-thiol, un sel du 1-[2-(diméthylamino)éthyl]-1H-tétrazole-5-thiol, le 3-Amino-5-methylthio-1 H-1,2,4-triazole, un sel du 3-Amino-5-methylthio-1H-1,2,4-triazole, le 5-(méthylthio)-1H-tétrazole, un sel du 5-(méthylthio)-1H-tétrazole, le 5-(éthylthio)-1H-tétrazole, un sel du 5-(éthylthio)-1H-tétrazole, le 1-méthyl-5-(méthylthio)-1H-tétrazole, un sel du 1-méthyl-5-(méthylthio)-1H-tétrazole, le 4-phényl-4H-1,2,4-triazole-3-thiol, un sel du 4-phényl-4H-1,2,4-triazole-3-thiol, le 5-méthyl-4H-1,2,4-triazole-3-thiol, un sel du 5-méthyl-4H-1,2,4-triazole-3-thiol, la 5-(trifluorométhyl)-2,4-dihydro-3H-1,2,4-triazole-3-thione, un sel de la 5-(trifluorométhyl)-2,4-dihydro-3H-1,2,4-triazole-3-thione, le 4-isopropyl-4H-1,2,4-triazole-3-thiol et un sel du 4-isopropyl-4H-1,2,4-triazole-3-thiol.

L'amine peut être choisie parmi le groupe contenant : la N,N,N',N',N"-pentaméthyldiéthylènetriamine, la pipérazine, la monoéthanolamine, la diéthanolamine, la méthyldiéthanolamine, la diisopropanolamine, la diglycolamine, le 2-amino-2-méthylpropanol-1, un sel de la glycine et un sel de la taurine.

Dans le cas où l'amine est choisie parmi la monoéthanolamine, la diéthanolamine et le 2-amino-2-méthylpropanol-1, le composé inhibiteur de dégradation peut être choisi parmi le 4-méthyl-4H-1,2,4-triazole-3-thiol, le 1H-1,2,4-triazole-3-thiol, le 1H-5-mercapto-1,2,3-triazole, le 5-mercapto-1-méthyltétrazole, le 5-méthyl-4H-1,2,4-triazole-3-thiol, le 4-isopropyl-4H-1,2,4-triazole-3-thiol, le 4-phényl-4H-1,2,4-triazole-3-thiol, le 1-[2-(diméthylamino)éthyl]-1H-tétrazole-5-thiol, le sel de sodium du 4-méthyl-4H-1,2,4-triazole-3-thiol, le sel de sodium du 1H-1,2,4-triazole-3-thiol, le sel de sodium du 1H-5-mercapto-1,2,3-triazole, le sel de sodium du 5-mercapto-1-méthyltétrazole, le sel de sodium du 5-méthyl-4H-1,2,4-triazole-3-thiol, le sel de sodium du 4-isopropyl-4H-1,2,4-triazole-3-thiol, le sel de sodium du 4-phényl-4H-1,2,4-triazole-3-thiol, le sel de potassium du 5-méthyl-4H-1,2,4-triazole-3-thiol, le sel de potassium du 4-méthyl-4H-1,2,4-triazole-3-thiol, le sel de potassium du 1H-1,2,4-triazole-3-thiol, le sel de potassium du 4-isopropyl-4H-1,2,4-triazole-3-thiol, le sel de potassium du 4-phényl-4H-1,2,4-triazole-3-thiol et le sel de potassium du 5-mercapto-1-méthyltétrazole.

La solution absorbante peut comporter au moins 39% poids de monoéthanolamine.

La présente invention décrit également un procédé pour absorber des composés acides contenus dans un effluent gazeux, dans lequel on met en contact l'effluent gazeux avec une solution aqueuse comportant au moins une amine, et dans lequel on contrôle la dégradation de ladite amine en introduisant au moins un composé inhibiteur de dégradation dérivé d'un triazole ou d'un tétrazole, comportant au moins un substituant comportant un atome de soufre.

Dans le procédé selon l'invention, la solution aqueuse peut être mise en oeuvre pour absorber des composés acides contenus dans l'un des effluents du groupe contenant le gaz naturel, les fumées de combustion, les gaz de synthèse, les gaz de raffinerie, les gaz obtenus en queue du procédé Claus, les gaz de fermentation de biomasse, les

gaz de cimenterie et les fumées d'incinérateur.

L'effluent gazeux peut comporter au moins 500ppm volumique d'oxygène.

On peut ajouter dans la solution aqueuse au moins un composé inhibiteur de dégradation choisi parmi le groupe contenant: le 1 H-1,2,4-triazole-3-thiol, un sel du 1H-1,2,4-triazole-3-thiol, le 5-phényl-1H-1,2,4-triazole-3-thiol, un sel du 5-phényl-1H-1,2,4-triazole-3-thiol, le 5-(4-pyridyl)-1H-1,2,4-triazole-3-thiol, un sel du 5-(4-pyridyl)-1H-1,2,4-triazole-3-thiol, le 5-(3-pyridyl)-1H-1,2,4-triazole-3-thiol, un sel du 5-(3-pyridyl)-1H-1,2,4-triazole-3-thiol, le 4-méthyl-4H-1,2,4-triazole-3-thiol, un sel du 4-méthyl-4H-1,2,4-triazole-3-thiol, le 4-méthyl-5-(2-thienyl)-4H-1,2,4-triazole-3-thiol, un sel du 4-méthyl-5-(2-thienyl)-4H-1,2,4-triazole-3-thiol, le 4-méthyl-5-(3-thienylméthyl)-4H-1,2,4-triazole-3-thiol, un sel du 4-méthyl-5-(3-thienylméthyl)-4H-1,2,4-triazole-3-thiol, le 4-cyclohexyl-5-sulfanyl-4H-1,2,4-triazol-3-ol, un sel du 4-cyclohexyl-5-sulfanyl-4H-1,2,4-triazol-3-ol, le 3-amine-1,2,4-triazole-5-thiol, un sel du 3-amino-1,2,4-triazole-5-thiol, le 4-amino-4H-1,2,4-triazole-3,5-dithiol, un sel du 4-amino-4H-1,2,4-triazole-3,5-dithiol, le [1,2,4]triazolo[4,3-a]pyridine-3-thiol, un sel du [1,2,4]triazolo[4,3-a]pyridine-3-thiol, le 1H-5-mercapto-1,2,3-triazole, un sel du 1H-5-mercapto-1,2,3-triazole, le 1-méthyl-1 H-tétrazole-5-thiol, un sel du 1-méthyl-1H-tétrazole-5-thiol, le 1-éthyl-1H-tétrazole-5-thiol, un sel du 1-éthyl-1H-tétrazole-5-thiol, le 1-phényl-1H-tétrazole-5-thlol, un sel du 1-phényl-1H-tétrazole-5-thiol, le 1-(4-hydroxyphényl)-1H-tétrazole-5-thiol, un sel du 1-(4-hydroxyphényl)-1H-tétrazole-5-thiol, l'acide 5-mercapto-1-tétrazoleacétique, un sel de l'acide 5-mercapto-1-tétrazoleacétique, le 1-[2-(diméthylamino)éthyl]-1H-tétrazole-5-thiol, un sel du 1-[2-(diméthylamino)éthyl]-1H-tétrazole-5-thiol, le 3-Amino-5-methylthio-1H-1,2,4-triazole, un sel du 3-Amino-5-methylthio-1H-1,2,4-triazole, le 5-(méthylthio)-1H-tétrazole, un sel du 5-(méthylthio)-1H-tétrazole, le 5-(éthylthio)-1H-tétrazole, un sel du 5-(éthylthio)-1H-tétrazole, le 1-méthyl-5-(méthylthio)-1H-tétrazole, un sel du 1-méthyl-5-(méthylthio)-1 H-tétrazole, le 4-phényl-4H-1,2,4-triazole-3-thiol, un sel du 4-phényl-4H-1,2,4-triazole-3-thiol, le 5-méthyl-4H-1,2,4-triazole-3-thiol, un sel du 5-méthyl-4H-1,2,4-triazole-3-thiol, la 5-(trifluorométhyl)-2,4-dihydro-3H-1,2,4-triazole-3-thione, un sel de la 5-(trifluorométhyl)-2,4-dihydro-3H-1,2,4-triazole-3-thione, le 4-isopropyl-4H-1,2,4-triazole-3-thiol et un sel du 4-isopropyl-4H-1,2,4-triazole-3-thiol.

Pour limiter la dégradation de la monoéthanolamine, de la diéthanolamine ou du 2-amino-2-méthylpropanol-1 en solution aqueuse mise en oeuvre pour capter le $CO_2$ des fumées de combustion, on peut ajouter dans la solution aqueuse au moins un composé inhibiteur de dégradation choisi parmi le groupe contenant : le 4-méthyl-4H-1,2,4-triazole-3-thiol, le 1 H-1,2,4-triazole-3-thiol, le 1H-5-mercapto-1,2,3-triazole, le 5-mercapto-1-méthyltétrazole, le 5-méthyl-4H-1,2,4-triazole-3-thiol, le 4-isopropyl-4H-1,2,4-triazole-3-thiol, le 4-phényl-4H-1,2,4-triazole-3-thlol, le 1-[2-(diméthylamino)éthyl]-1H-tétrazole-5-thiol, le sel de sodium du 4-méthyl-4H-1,2,4-triazole-3-thiol, le sel de sodium du 1 H-1,2,4-triazole-3-thiol, le sel de sodium du 1H-5-mercapto-1,2,3-triazole, le sel de sodium du 5-mercapto-1-méthyltétrazole, le sel de sodium du 5-méthyl-4H-1,2,4-triazole-3-thiol, le sel de sodium du 4-isopropyl-4H-1,2,4-triazole-3-thiol, le sel de sodium du 4-phényl-4H-1,2,4-triazole-3-thiol, le sel de potassium du 5-méthyl-4H-1,2,4-triazole-3-thiol, le sel de potassium du 4-méthyl-4H-1,2,4-triazole-3-thiol, le sel de potassium du 1H-1,2,4-triazole-3-thiol, le sel de potassium du 4-isopropyl-4H-1,2,4-triazole-3-thiol, le sel de potassium du 4-phényl-4H-1,2,4-triazole-3-thiol et le sel de potassium du 5-mercapto-1-méthyltétrazole.

D'autres caractéristiques et avantages de l'invention seront mieux compris et apparaîtront clairement à la lecture de la description faite ci-après, en référence aux figures annexées dans lesquelles les figures 1 à 5 représentent la teneur en $NH_3$ dans la phase gaz traité par une solution absorbante en fonction de l'ajout ou non d'agents inhibiteurs de dégradation dans la solution absorbante et en fonction de la teneur en amine dans la solution absorbante.

Afin de réduire la dégradation d'une solution absorbante, les inventeurs ont montré que la dégradation d'une solution absorbante comportant des composés organiques munis d'une fonction amine en solution aqueuse est sensiblement réduite en présence d'une faible quantité d'agents inhibiteurs de dégradation décrits ci-après.

Les argents inhibiteurs de dégradation selon l'invention sont des composés appartenant à la famille des dérivés des triazoles ou d'un tétrazole dont au moins un substituant contient un atome de soufre.

Dans la présente description, on entend par triazoles les composés cycliques à 5 atomes dont le cycle comporte 3 atomes d'azote, 2 atomes de carbone et 2 insaturations et par tétrazole un composé cyclique à 5 atomes dont le cycle comporte 4 atomes d'azote, 1 atome de carbone et 2 insaturations.

Dans la présente description, on entend par dérivés des triazoles dont au moins un substituant contient un atome de soufre, que au moins un des atomes de carbone du cycle triazole soit lié à un atome de soufre par une simple liaison.

Dans la présente description, on entend par dérivés d'un tétrazole dont au moins un substituant contient un atome de soufre, que l'atome de carbone du cycle térazole soit lié à un atome de soufre par une simple liaison.

Les composés inhibiteur de dégradation selon l'invention peuvent par exemple répondre aux formules générales suivantes :

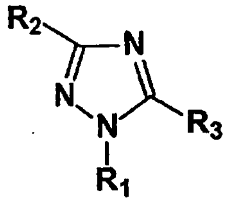
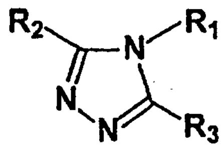
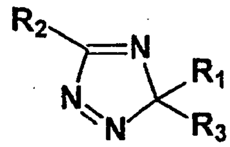

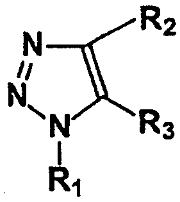
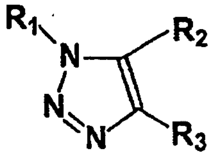
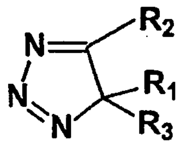

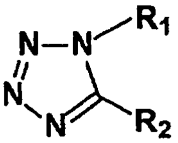
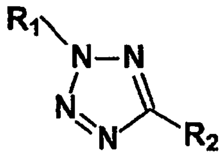
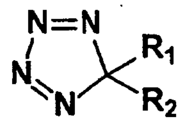

Le radical $R_1$ est choisi parmi :

- ○ un atome d'hydrogène,
- ○ un radical hydrocarboné comprenant de 1 à 12 atomes de carbone, saturé ou non, linéaire, branché ou cyclique, hétérocyclique ou aromatique pouvant éventuellement contenir des hétéroatomes, des halogènes, et pouvant renfermer des fonctions hydroxyles, cétones, carboxyliques ou nitriles,
- ○ un radical amino de formule générale $-NR_4R_5$ avec $R_4$ et $R_5$ indépendamment choisis parmi :
  - ○ un atome d'hydrogène
  - ○ un radical hydrocarboné comprenant 1 à 12 atomes de carbone, de préférence de 1 à 3 atomes de carbone, pouvant éventuellement contenir des hétéroatomes, des halogènes, et pouvant renfermer des fonctions hydroxyles, cétones, carboxyliques ou nitriles.

Chacun des radicaux $R_2$ et $R_3$ est choisi indépendamment parmi les éléments suivants :

a) -S-X
dans lequel X est choisi parmi :

- ○ un atome d'hydrogène,
- ○ un élément alcalin ou alcalino-terreux,
- ○ un métal monovalent ou multivalent,
- ○ un cation ammonium $NH_4^+$ ou résultant de la protonation d'une fonction amine,
- ○ un cation phosphonium,
- ○ un radical hydrocarboné comprenant de 1 à 12 atomes de carbone, saturé ou non, linéaire, branché ou cyclique, hétérocyclique ou aromatique pouvant éventuellement contenir des hétéroatomes, des halogènes, et pouvant renfermer des fonctions hydroxyles, cétones, carboxyliques, amines ou nitriles,
- ○ selon un autre mode de réalisation de l'invention, X peut également être un radical thiyl-triazole ou thio-triazole ou un radical thiyl-tétrazole ou thio-tétrazole substitué ou non. Dans ce cas, le composé inhibiteur de dégradation selon l'invention comprend deux hétérocycles choisis parmi le triazole et le tétrazole, les deux hétérocycles étant reliés entre eux par deux atomes de soufre.

b) un atome d'hydrogène,
c) un radical hydroxyle,
d) un radical amino de formule générale $-NR_4R_5$ avec $R_4$ et $R_5$ indépendamment choisis parmi :

○ un atome d'hydrogène,

○ un radical hydrocarboné comprenant de 1 à 12 atomes de carbone, de préférence de 1 à 3 atomes de carbone pouvant éventuellement contenir des hétéroatomes, des halogènes, et pouvant renfermer des fonctions hydroxyles, cétones, carboxyliques ou nitriles,

e) un radical comprenant 1 à 12 atomes de carbone, saturé ou non, linéaire, branché ou cyclique, hétérocyclique ou aromatique pouvant éventuellement contenir des hétéroatomes, des halogènes, et pouvant renfermer des fonctions hydroxyles, cétones, carboxyliques ou nitriles.

Le fait que le composé inhibiteur de dégradation selon l'invention appartient à la famille des dérivés des triazoles ou d'un tétrazole dont au moins un substituant contient un atome de soufre se traduit par le fait que dans les formules générales mentionnées ci-dessus, au moins R2 ou R3 répond à la définition de - S-X. En particulier dans le cas des dérivés du tétrazole, R2 répond à la définition de -S-X.

Dans le cas où X est un élément alcalino-terreux ou métallique multivalent, il est entendu que pour respecter la neutralité des molécules, elles pourront prendre les formes leur permettant de respecter cette neutralité dans le respect des règles de la chimie et que l'on peut par exemple illustrer ainsi sur une des formules de l'invention :

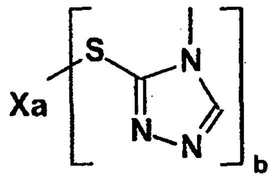

avec a et b étant des nombres entiers permettant de respecter la neutralité dans le respect des règles de la chimie.

Selon un premier mode de réalisation, dans la définition des formules générales des composés inhibiteurs de dégradation selon l'invention, $R_1$, $R_2$, $R_3$, $R_4$, $R_5$ et X sont indépendants, c'est-à-dire qu'ils ne sont pas reliés entre eux. Cependant, selon un deuxième mode de réalisation, dans le cas où deux éléments parmi $R_1$, $R_2$, $R_3$, $R_4$, $R_5$ et X sont des radicaux hydrocarbonés, ils peuvent être reliés par une liaison covalente pour former un cycle ou un hétérocycle aromatique ou non constitué de 5, 6, 7 ou 8 atomes dans le respect des règles de la chimie.

Les molécules de l'invention peuvent aussi exister sous leur forme dite tautomère ou mésomère lorsque cela est permis, et ce, dans le respect des règles de la chimie organique.

Les solutions absorbantes selon l'invention peuvent être mises en oeuvre pour désacidifier les effluents gazeux suivants : le gaz naturel, les gaz de synthèse, les fumées de combustion, les gaz de raffinerie, les gaz obtenus en queue du procédé Claus, les gaz de fermentation de biomasse, les gaz de cimenterie, les fumées d'incinérateur. Ces effluents gazeux contiennent un ou plusieurs des composés acides suivants : le $CO_2$, l'$H_2S$, des mercaptans, du COS, du $SO_2$, du $NO_2$, du $CS_2$. En particulier, le procédé selon l'invention, peut être mis en oeuvre pour absorber des composés acides contenus dans un effluent gazeux contenant de l'oxygène, comme par exemple les fumées de combustion. La teneur en oxygène dans l'effluent gazeux peut être supérieure à 500ppm en volume, de préférence supérieure à 0,5%, voire au moins 1%, 3% ou 5% volumique. En général, la teneur en oxygène dans l'effluent gazeux reste inférieure à 20% en volume. Les fumées de combustion sont produites notamment par la combustion d'hydrocarbures, de biogaz, de charbon dans une chaudière ou pour une turbine à gaz de combustion, par exemple dans le but de produire de l'électricité. Ces fumées peuvent comporter entre 50 % et 90 % d'azote, entre 5 % et 20 % de dioxyde de carbone. Les fumées comportent en général au moins 500ppm volumique, de préférence au moins 1% volumique, voire 2%, 3% ou 5% volumique d'oxygène, jusqu'à une teneur qui en général n'excède pas 20 % volume d'oxygène.

La mise en oeuvre d'une solution absorbante pour désacidifier un effluent gazeux est généralement réalisée en effectuant une étape d'absorption suivie d'une étape de régénération. L'étape d'absorption consiste à mettre en contact l'effluent gazeux avec la solution absorbante. Lors du contact, les composés organiques munis d'une fonction amine de la solution absorbante réagissent avec les composés acides contenus dans l'effluent de manière à obtenir un effluent gazeux appauvri en composés acides et une solution absorbante enrichie en composés acides. L'étape de régénération consiste notamment à chauffer et, éventuellement à détendre, au moins une partie de la solution absorbante enrichie en composés acides afin de libérer les composés acides sous forme gazeuse. La solution absorbante régénérée, c'est-à-dire appauvrie en composés acides est recyclée à l'étape d'absorption.

La solution absorbante selon l'invention comporte des composés organiques en solution aqueuse. De manière générale, les composés organiques sont des amines, c'est-à-dire qu'ils comportent au moins une fonction amine. Les composés organiques peuvent être en concentration variable par exemple compris entre 10% et 99% poids, de préférence entre 20% et 75% poids, voire entre 20% et 50% poids, dans la solution aqueuse. La solution absorbante peut contenir entre 1% et 90% poids d'eau, de préférence entre 25% et 80% poids, voire entre 50% et 70% poids d'eau.

Par exemples les composés organiques sont des amines tels que la N,N,N',N',N"-pentaméthyldiéthylènetriamine ou la pipérazine. Par exemple la pipérazine est utilisée pour le traitement du gaz naturel et pour la décarbonatation des fumées de combustion.

Les composés organiques peuvent également être des alcanolamines telles que la monoéthanolamine (MEA), la diéthanolamine (DEA), la méthyldiéthanolamine (MDEA), la diisopropanolamine (DIPA), la diglycolamine ou le 2-amino-2-méthylpropanol-1 (AMP). De préférence, la MDEA et la DEA sont couramment utilisées pour la désacidification du gaz naturel. La MEA et l'AMP sont plus particulièrement utilisées pour la décarbonatation des fumées de combustion.

Les composés organiques peuvent également être des sels d'acides aminés tels que les sels de la glycine ou de la taurine qui sont notamment mis en oeuvre pour le captage du $CO_2$ dans les fumées de combustion.

En outre, la solution absorbante selon l'invention peut contenir des composés qui absorbent physiquement au moins partiellement un ou plusieurs composés acides de l'effluent gazeux. Par exemple la solution absorbante peut comporter entre 5% et 50% poids de composés absorbants à caractère physique tel que du méthanol, du sulfolane ou de la N-formyl morpholine.

Un autre avantage de l'invention réside dans le fait que l'utilisation d'agents inhibiteurs de dégradation selon l'invention permet d'augmenter la concentration en amines communément utilisée par l'homme du métier et d'augmenter ainsi les performances du procédé : augmentation de la capacité et de la vitesse d'absorption des composés acides par la solution absorbante entraînant une réduction des coûts d'investissement et des coûts opératoires de l'unité industrielle. En effet, comme montré ci-après dans l'exemple 3, en absence d'agents inhibiteurs de dégradation, la vitesse de dégradation des amines augmente avec l'augmentation de la concentration en amines. Ainsi dans le cas par exemple de l'utilisation d'une solution aqueuse de MEA (monoéthanolamine) pour le captage du $CO_2$ dans les fumées de combustion, la concentration en MEA est communément limitée à 30% poids pour limiter la dégradation de cette amine. Il est entendu ici que la concentration en amine est définie en pourcentage poids dans l'eau avant absorption de $CO_2$. Ainsi par exemple, une solution absorbante utilisée pour le captage du $CO_2$ dans une fumée de combustion et contenant un agent inhibiteur de dégradation selon l'invention peut contenir plus de 30% poids et de préférence plus de 35% poids de MEA, une bonne valeur de la concentration en MEA étant au moins égale à 39% poids.

Parmi l'ensemble des molécules appartenant à la famille des dérivés des triazoles et d'un tétrazole dont au moins un substituant contient un atome de soufre, on utilise de préférence les agents inhibiteurs de dégradation suivants :

le 1H-1,2,4-triazole-3-thiol, le 5-phényl-1H-1,2,4-triazole-3-thiol, le 5-(4-pyridyl)-1H-1,2,4-triazole-3-thiol, le 5-(3-pyridyl)-1H-1,2,4-triazole-3-thiol, le 4-méthyl-4H-1,2,4-triazole-3-thiol, le 4-méthyl-5-(2-thienyl)-4H-1,2,4-triazole-3-thiol, le 4-méthyl-5-(3-thienylméthyl)-4H-1,2,4-triazole-3-thiol, le 4-cyclohexyl-5-sulfanyl-4H-1,2,4-triazol-3-ol, le 3-amino-1,2,4-triazole-5-thiol, le 4-amino-4H-1,2,4-triazole-3,5-dithiol, le [1,2,4]triazolo[4,3-a]pyridine-3-thiol, le 1H-5-mercapto-1,2,3-triazole, le 1-méthyl-1 H-tétrazole-5-thiol, le 1-éthyl-1 H-tétrazole-5-thiol, le 1-phényl-1 H-tétrazole-5-thiol, le 1-(4-hydroxyphényl)-1H-tétrazole-5-thiol, l'acide 5-mercapto-1-tétrazoleacétique, le 1-[2-(diméthylamino) éthyl]-1H-tétrazole-5-thiol, le 3-Amino-5-methytthio-1H-1,2,4-triazole, le 5-(méthylthio)-1H-tétrazole, le 5-(éthylthio)-1H-tétrazole et le 1-méthyl-5-(méthylthio)-1H-tétrazole, le 4-phényl-4H-1,2,4-triazole-3-thiol, le 5-méthyl-4H-1,2,4-triazole-3-thiol, la 5-(trifluorométhyl)-2,4-dihydro-3H-1,2,4-triazole-3-thione qui est une forme mésomère du 5-(trifluorométhyl)-4H-1,2,4,-triazole-3-thiol, le 4-isopropyl-4H-1,2,4-triazole-3-thiol, ainsi que les sels des éléments précédemment cités.

Les sels des composés inhibiteurs de dégradation selon l'invention peuvent être obtenus par exemple par leur neutralisation à l'aide d'un hydroxyde ou un carbonate alcalin (de préférence un hydroxyde ou un carbonate de sodium ou de potassium), alcalinoterreux ou métallique ou d'ammonium ou à l'aide d'une amine présente dans la solution absorbante. Dans ce cas, les fonctions présentant un caractère acide, telles que par exemple les fonctions thiols et par exemple les éventuelles fonctions acides carboxyliques, peuvent être partiellement ou totalement neutralisées.

Les sels des composés inhibiteurs de dégradation selon l'invention peuvent être également obtenus par exemple par leur neutralisation à l'aide d'un acide organique ou inorganique. Dans ce cas, c'est au moins un atome d'azote présent sur la molécule qui est protoné.

Les composés inhibiteurs de dégradation listés au paragraphe précédent sont particulièrement bien adaptés à la prévention de la dégradation d'amine en solution aqueuse mise en oeuvre dans un procédé de captage du $CO_2$ contenu dans des fumées de combustion.

Pour limiter la dégradation d'une solution absorbante composée d'amine, en particulier d'alcanolamine, par exemple la monoéthanolamine (MEA), la diéthanolamine (DEA) ou le 2-amino-2-méthylpropanol-1 (AMP), en solution aqueuse notamment pour capter le $CO_2$ des fumées de combustion ou pour désacidifier un gaz naturel, on peut utiliser de préférence l'un des composés suivants :

- le 4-méthyl-4H-1,2,4-triazole-3-thiol

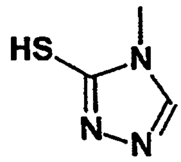

- le 1H-1,2,4-triazole-3-thiol

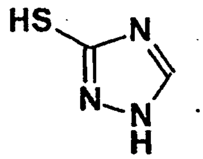

- le 1H-5-mercapto-1,2,3-triazole

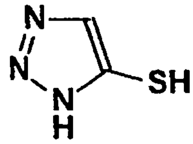

- le 5-mercapto-1-méthyltétrazole, appelé aussi 1-méthyl-1H-tétrazole-5-thiol

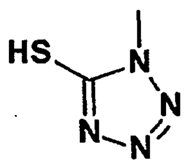

- le 5-méthyl-4H-1,2,4-triazole-3-thiol

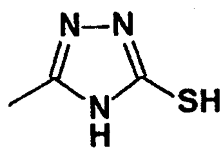

- le 4-isopropyl-4H-1,2,4-triazole-3-thiol

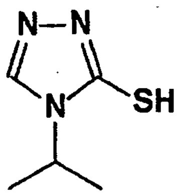

- le 4-phényl-4H-1,2,4-triazole-3-thiol

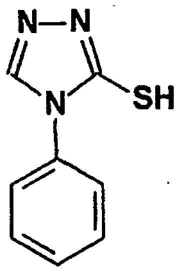

- le 1-[2-(diméthylamino)éthyl]-1H-tétrazole-5-thiol

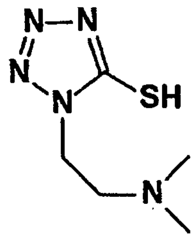

ainsi que leurs sels tels par exemple les sels de sodium, de potassium ou d'ammonium, comme par exemple :

- le sel de sodium du 4-méthyl-4H-1,2,4-triazole-3-thiol

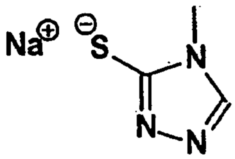

- le sel de sodium du 1H-1,2,4-triazole-3-thiol

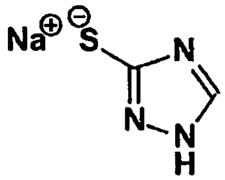

- le sel de sodium du 1H-5-mercapto-1,2,3-triazole

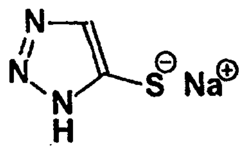

- le sel de sodium du 5-mercapto-1-méthyltétrazole, appelé aussi sel de sodium du 1-méthyl-1H-tétrazole-5-thiol

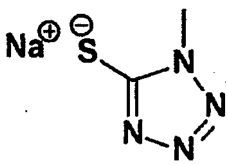

- le sel de sodium du 5-méthyl-4H-1,2,4-triazole-3-thiol

- le sel de sodium du 4-isopropyl-4H-1,2,4-triazole-3-thiol
- le sel de sodium du 4-phényl-4H-1,2,4-triazole-3-thiol
- le sel de potassium du 5-méthyl-4H-1,2,4-triazole-3-thiol
- le sel de potassium du 4-méthyl-4H-1,2,4-triazole-3-thiol
- le sel de potassium du 1H-1,2,4-triazole-3-thiol
- le sel de potassium du 4-isopropyl-4H-1,2,4-triazole-3-thiol
- le sel de potassium du 4-phényl-4H-1,2,4-triazole-3-thiol
- le sel de potassium du 5-mercapto-1-méthyltétrazole, appelé aussi sel de potassium du 1-méthyl-1H-tétrazole-5-thiol.

De préférence, selon l'invention, on utilise le sel de sodium du 1H-1,2,4-triazole-3-thiol, le sel de sodium du 4-méthyl-4H-1,2,4-triazole-3-thiol, le 4-méthyl-4H-1,2,4-triazole-3-thiol, le 5-méthyl-4H-1,2,4-triazole-3-thiol, le 4-isopropyl-4H-1,2,4-triazole-3-thiol, le 4-phényl-4H-1,2,4-triazole-3-thiol, le 1-méthyl-1H-tétrazole-5-thiol ou le 1-[2-(diméthylamino)éthyl]-1H-tétrazole-5-thiol pour limiter la dégradation d'une amine, en particulier la MEA, en solution aqueuse mise en oeuvre pour désacidifier un effluent gazeux, notamment dans le cadre du captage de $CO_2$ contenu dans des fumées de combustion.

De préférence, selon l'invention, on utilise le 4-méthyl-4H-1,2,4-triazole-3-thiol pour limiter la dégradation d'une amine, en particulier la DEA, en solution aqueuse mise en oeuvre pour désacidifier un effluent gazeux, notamment dans le cadre de la désacidification du gaz naturel.

De préférence, selon l'invention, on utilise le 4-méthyl-4H-1,2,4-triazole-3-thiol pour limiter la dégradation d'une amine, en particulier la AMP, en solution aqueuse mise en oeuvre pour désacidifier un effluent gazeux, notamment dans le cadre du captage de $CO_2$ contenu dans des fumées de combustion.

La solution absorbante selon l'invention comporte une quantité d'agents inhibiteurs de dégradation définis par la formule générale décrite ci-dessus. La solution absorbante peut comporter un ou plusieurs agents inhibiteurs de dégradation différents correspondant à ladite formule générale. De plus, dans la solution absorbante, les agents inhibiteurs de dégradation selon l'invention peuvent être associés à d'autres composés inhibiteurs de dégradation de familles chimiques différentes. Selon l'invention, la solution absorbante comporte entre 5 ppm et 5% poids d'agents inhibiteurs de dégradation selon l'invention, de préférence de 50 ppm à 2% poids, et une excellente teneur en agents inhibiteurs de dégradation dans la solution étant comprise entre 100 ppm et 1% poids.

Les exemples présentés ci-après permettent de comparer et d'illustrer les performances des agents inhibiteurs de dégradation selon l'invention, en terme de réduction de la dégradation des amines en solution aqueuse, de réduction des émissions de composés de dégradation volatils et de possibilité d'augmenter la concentration en amines sans augmenter leur dégradation.

EXEMPLE 1 :

Les amines de la solution absorbante peuvent être dégradées dans une utilisation selon l'invention engendrant une consommation de l'amine.

Les essais de dégradation d'une amine en solution aqueuse sont effectués selon le mode opératoire suivant.

100g de solution de MEA (monoéthanolamine) 30% poids dans l'eau désionisée sont placés dans un réacteur en verre surmonté d'un condenseur pour éviter l'évaporation de l'eau. Le réacteur est chauffé à 80°C dans un bloc chauffant électrique. La solution est agitée à 1000 tours par minute par un barreau aimanté. La présence de contre pales empêche la formation d'un vortex. Un gaz est mis en contact avec la solution à l'aide d'un tube plongeant pendant 7 jours à pression atmosphérique. Selon les essais, on fait varier la nature du gaz mis en contact avec la solution. De même les essais sont conduits soit en absence soit en présence de différents agents inhibiteurs de dégradation incorporés dans la solution aqueuse d'amine à 0,25% poids.

Lorsque l'essai est conduit uniquement en présence de $CO_2$ et en l'absence d'oxygène, le gaz mis en contact avec la solution est un mélange de 7Nl/h d'azote et de 0,033 Nl/h de $CO_2$ réalisé dans une chambre de mélange. Dans ce cas, le gaz comporte uniquement du $CO_2$ et de l'azote.

Lorsque l'essai est conduit en présence de $CO_2$ et d'oxygène, le gaz mis en contact avec la solution est un mélange de 7Nl/h d'air atmosphérique, c'est-à-dire de l'air ambiant non purifié, et de 0,033 Nl/h de $CO_2$ réalisé dans une chambre de mélange. Dans ce cas, le gaz contient du $CO_2$, de l'azote et de l'oxygène, la teneur en oxygène dans le gaz étant de 21% environ.

Une analyse par chromatographie en phase gazeuse de la solution ainsi dégradée est réalisée à la fin de l'essai. La méthode chromatographique utilise une colonne polaire, un gaz vecteur, l'hélium, un étalon interne, le triéthylèneglycol et une détection FID (Flame Induced Detection). Cette analyse permet de déterminer la concentration résiduelle de MEA et donc le taux dégradation défini par :

$$taux_de_dégradation = \left(1 - \frac{[MEA]finale}{[MEA]initiale}\right) * 100$$

Le tableau 1 donne les taux de dégradation d'une solution aqueuse de MEA (monoéthanolamine) 30% poids, en présence d'un agent inhibiteur de dégradation ou non et soumise à un gaz renfermant de l'azote, du $CO_2$ et contenant ou non de oxygène :

- Cas n°1 : sans oxygène et sans additif
- Cas n°2 : en présence d'oxygène et sans additif
- Cas n°3 : en présence d'oxygène et en présence d'un additif antioxygène conventionnel, le sulfite de sodium ($Na_2SO_3$).
- Cas n°4 : en présence d'oxygène et en présence d'un additif selon l'invention, le sel de sodium du 1H-1,2,4-triazole-3-thiol
- Cas n°5 : en présence d'oxygène et en présence d'un additif selon l'invention, le sel de sodium du 4-méthyl-4H-1,2,4-triazole-3-thiol

Tableau 1 : comparaison des taux de dégradation de la MEA 30% poids obtenus dans l'eau à 80°C dans les différents cas.

| CAS | Teneur en $O_2$ | Nom de l'additif | Taux de dégradation |
|---|---|---|---|
| 1 | 0% | - | <3% |
| 2 | 21% | - | 70% |
| 3 | 21% | sulfite de sodium ($Na_2SO_3$) | 71% |
| 4 | 21% | sel de sodium du 1H-1,2,4-triazote-3-thiol | <3% |
| 5 | 21% | sel de sodium du 4-méthyl-4H-1,2,4-triazole-3-thiol | <3% |

Il apparaît clairement que :

1. la solution de MEA n'est pas dégradée en présence du seul $CO_2$ en l'absence d'oxygène
2. la dégradation de la MEA est attribuable à la présence d'oxygène
3. en présence d'un agent antioxygène conventionnel réducteur tel le sulfite de sodium, la dégradation de la MEA en présence d'oxygène n'est pas diminuée
4. en présence d'additifs selon l'invention, la dégradation de la MEA est ramenée au même niveau que celle constatée en l'absence d'oxygène, c'est-à-dire considérée comme nulle car inférieure à l'incertitude de la mesure qui est de 3%.

En conclusion, les additifs selon invention combattent efficacement l'effet de l'oxygène sur la dégradation de la MEA.

EXEMPLE 2 :

En particulier, les amines peuvent être dégradées par l'oxygène engendrant la formation de produits volatils, qui sont entraînés dans les effluents gazeux du procédé. Ainsi, par exemple dans le cas du traitement de fumées en post-combustion dans un procédé utilisant une solution aqueuse de MEA des quantités importantes d'ammoniac sont formées. L'ammoniac ainsi formé est entraîné dans l'atmosphère avec les fumées traitées ce qui pose des problèmes quant à la protection de l'environnement.

La figure 1 présente un suivi de la concentration en ammoniac dans le gaz sortant du réacteur dans les cas 2, 3, 4 et 5 définis dans l'exemple 1. [A] correspond à la concentration en ammoniac en ppm volume dans le gaz de sortie du réacteur, t représente le temps exprimé en jours. Les cas 2, 3, 4 et 5 sont représentés respectivement par la courbe 2 en trait continu, la courbe 3 avec des carrés, la courbe 4 avec des triangles et la courbe 5 avec des croix.

La concentration en ammoniac dans le gaz sortant du réacteur est déterminée par une analyse en ligne par

spectrométrie Infra-Rouge à Transformée de Fourrier.

Dans le cas des agents inhibiteurs de dégradation selon l'invention, la teneur en $NH_3$ est inférieure à 10ppm pendant toute la durée du test (voir courbes 4 et 5) alors qu'elle atteint près de 2000ppm dans le cas d'un agent antioxygène conventionnel (voir courbe 3), le sulfite de sodium et près de 2500ppm sans agent inhibiteur de dégradation (voir courbe 2).

Cet exemple montre donc bien que les agents inhibiteurs de dégradation selon l'invention sont efficaces pour réduire les émissions de composés de dégradation volatils. Par conséquent, dans un procédé industriel utilisant une solution absorbante contenant des agents inhibiteurs de dégradation selon l'invention, les émissions de composés volatils en tête d'absorbeur seront bien moindres qu'en absence d'agents inhibiteurs de dégradation.

EXEMPLE 3 :

Cet exemple montre que l'utilisation des agents inhibiteurs de dégradation de l'amine permet l'augmentation de la concentration en amine sans augmentation de la dégradation. Cet exemple donne les résultats obtenus avec une solution aqueuse de MEA à 40% poids.

Les essais de dégradation d'une amine en solution aqueuse sont effectués selon le mode opératoire suivant.

100g de solution de MEA 40% poids dans l'eau désionisée sont placés dans un réacteur en verre surmonté d'un condenseur pour éviter l'évaporation de l'eau. Le réacteur est chauffé à 80°C dans un bloc chauffant électrique. La solution est agitée à 1000 tours par minute par un barreau aimanté. La présence de contre pales empêche la formation d'un vortex. Un gaz est mis en contact avec la solution à l'aide d'un tube plongeant pendant 7 jours à pression atmosphérique. Les essais sont conduits soit en absence soit en présence d'un agent inhibiteur de dégradation incorporé dans la solution aqueuse d'amine à 0,25% poids.

L'essai est conduit en présence de $CO_2$ et d'oxygène : le gaz mis en contact avec la solution est un mélange de 7Nl/h d'air atmosphérique, c'est-à-dire de l'air ambiant non purifié, et de 0,033 Nl/h de $CO_2$ réalisé dans une chambre de mélange. Le gaz contient donc du $CO_2$, de l'azote et de l'oxygène, la teneur en oxygène dans le gaz étant de 21 % environ.

Une analyse par chromatographie en phase gazeuse de la solution ainsi dégradée est réalisée à la fin de l'essai. La méthode chromatographique utilise une colonne polaire, un gaz vecteur, l'hélium, un étalon interne, le triéthylèneglycol et une détection FID (Flame Induced Detection). Cette analyse permet de déterminer la concentration résiduelle de MEA.

Une vitesse moyenne de dégradation sur la durée de l'essai peut donc être calculée :

$$vitesse_moyenne_de_dégradation = \frac{[MEA]_{initiale} - [MEA]_{finale}}{durée_essai} * masse_de_solution$$

De même un taux dégradation peut être calculé :

$$taux_de_dégradation = \left(1 - \frac{[MEA]finale}{[MEA]initiale}\right) * 100$$

Le tableau 2 présente les vitesses moyennes de dégradation de la MEA obtenues dans les mêmes conditions dans le cas n°6 d'une MEA 40% poids sans additif et le cas n°2 d'une MEA 30% poids sans additif défini dans l'exemple 1.

Tableau 2 : comparaison des vitesses moyennes de dégradation de la MEA 30% et 40% poids en absence d'un agent inhibiteur de dégradation selon l'invention.

| CAS | Teneur en $O_2$ | [MEA] en % poids | Vitesse moyenne de dégradation (g/jour) |
|---|---|---|---|
| 2 | 21% | 30% | 2,99 |
| 6 | 21% | 40% | 3,72 |

Le tableau 2 confirme bien qu'une solution aqueuse de MEA 40% poids se dégrade plus vite qu'une solution de MEA 30% poids. Ainsi pour une même durée, la masse de MEA dégradée est plus importante dans le cas d'une

solution aqueuse de MEA 40% poids.

Le tableau 3 donne les taux de dégradation d'une solution aqueuse de MEA 40% poids, en présence d'un agent inhibiteur de dégradation ou non :

- Cas n°6 : sans additif
- Cas n°7 : en présence d'un additif selon l'invention, le sel de sodium du 4-méthyl-4H-1,2,4-triazole-3-thiol
- Cas n°8 : en présence d'un additif selon l'invention, le 4-méthyl-4H-1,2,4-triazole-3-thiol
- Cas n°9 : en présence d'un additif selon l'invention, le 5-méthyl-4H-1,2,4-triazole-3-thiol
- Cas n°10 : en présence d'un additif selon l'invention, le 4-isopropyl-4H-1,2,4-triazole-3-thiol
- Cas n°11 : en présence d'un additif selon l'invention, le 4-phényl-4H-1,2,4-triazole-3-thiol
- Cas n°12 : en présence d'un additif selon l'invention, le 1-méthyl-1 H-tétrazole-5-thiol
- Cas n°13 : en présence d'un additif selon l'invention, le 1-[2-(diméthylamino)éthyl]-1H-tétrazole-5-thiol

Tableau 3 : comparaison des taux de dégradation de la MEA 40% poids obtenus dans l'eau à 80°C en absence et en présence d'un agent inhibiteur de dégradation selon l'invention.

| CAS | Teneur en $O_2$ | Nom de l'additif | Taux de dégradation |
|---|---|---|---|
| 6 | 21% | - | 66% |
| 7 | 21% | sel de sodium du 4-méthyl-4H-1,2,4-triazole-3-thiol | <3% |
| 8 | 21% | 4-méthyl-4H-1,2,4-triazole-3-thiol | <3% |
| 9 | 21% | 5-méthyl-4H-1,2,4-triazole-3-thiol | <3% |
| 10 | 21% | 4-isopropyl-4H-1,2,4-triazole-3-thiol | <3% |
| 11 | 21% | 4-phényl-4H-1,2,4-triazole-3-thiol | <3% |
| 12 | 21% | 1-méthyl-1H-tétrazole-5-thiol | <3% |
| 13 | 21% | 1-[2-(diméthylamino) éthyl]-1H-tétrazole-5-thiol | <3% |

Il apparaît clairement qu'en présence d'un additif selon l'invention, la dégradation de la MEA à 40 % poids dans l'eau peut être considérée comme nulle car inférieure à l'incertitude de la mesure qui est de 3%.

En conclusion, dans le cas de la MEA, les additifs selon l'invention permettent d'augmenter la concentration en amine communément utilisée par l'homme du métier sans augmenter la dégradation de l'amine.

EXEMPLE 4 :

Cet exemple montre que l'utilisation des agents inhibiteurs de dégradation de l'amine permet l'augmentation de la concentration en amine sans augmenter les émissions de produits de dégradation volatils. Cet exemple donne les résultats obtenus avec une solution aqueuse de MEA à 40% poids.

La figure 2 présente un suivi de la concentration en ammoniac dans le gaz sortant du réacteur dans les cas 6 et 7 définis dans l'exemple 3. [A] correspond à la concentration en ammoniac en ppm volume dans le gaz de sortie du réacteur, t représente le temps exprimé en jours. Les cas 6 et 7 sont représentés respectivement par la courbe 6 avec des ronds blancs et la courbe 7 avec des losanges noirs.

La figure 3 présente un suivi de la concentration en ammoniac dans le gaz sortant du réacteur dans les cas 8 à 13 définis dans l'exemple 3 et dans une nouvelle évaluation du cas 6. [A] correspond à la concentration en ammoniac en ppm volume dans le gaz de sortie du réacteur, t représente le temps exprimé en jours. Les cas 6, 8, 9, 10, 11, 12 et 13 sont représentés respectivement par la courbe 6 avec des ronds blancs, la courbe 8 avec des losanges blancs, la courbe 9 avec des triangles blancs, la courbe 10 avec des croix, la courbe 11 avec des carrés blancs, la courbe 12 avec des ronds noirs et la courbe 13 avec des carrés noirs.

La concentration en ammoniac dans le gaz sortant du réacteur est déterminée par une analyse en ligne par spectrométrie Infra-Rouge à Transformée de Fourrier.

Dans le cas des agents inhibiteurs de dégradation selon l'invention, la teneur en $NH_3$ est inférieure à 10ppm pendant toute la durée du test alors qu'elle dépasse rapidement les 2000ppm sans agent inhibiteur de dégradation. Il apparaît clairement qu'en présence d'un additif selon l'invention, les émissions d'ammoniac liées à la dégradation de la MEA à 40 % poids dans l'eau sont considérablement réduites.

Cet exemple montre donc bien, dans le cas de la MEA, que les agents inhibiteurs de dégradation selon l'invention permettent d'augmenter la concentration en amine communément utilisée par l'homme du métier sans augmenter les émissions d'ammoniac.

Par conséquent, dans un procédé industriel utilisant une solution absorbante contenant des agents inhibiteurs de dégradation selon l'invention, les émissions de composés volatils en tête d'absorbeur seront bien moindres qu'en absence d'agents inhibiteurs de dégradation même si la concentration en amine est augmentée par rapport à la concentration communément utilisée par l'homme du métier.

EXEMPLE 5 :

Les exemples précédents ont montré l'efficacité des agents inhibiteurs de dégradation selon l'invention pour réduire la dégradation des amines en particulier dans le cas de la MEA et ce même à 40% poids d'amine. Dans cet exemple on montre que les agents inhibiteurs de dégradation selon l'invention sont efficaces pour réduire la dégradation de la N,N-diéthanolamine (DEA), très utilisée par exemple dans le cas du traitement du gaz naturel et du 2-amino-2-méthylpropanol-1 (AMP), utilisé par exemple dans le cas du captage du $CO_2$ dans les fumées.

Cet exemple donne les résultats obtenus avec des solutions aqueuses de DEA à 40% poids et d'AMP à 40% poids.

Les essais de dégradation d'une amine en solution aqueuse sont effectués selon le mode opératoire suivant :

> 100g de solution d'amine 40% poids dans l'eau désionisée sont placés dans un réacteur en verre surmonté d'un condenseur pour éviter l'évaporation de l'eau. Le réacteur est chauffé à 80°C dans un bloc chauffant électrique. La solution est agitée à 1000 tours par minute par un barreau aimanté. La présence de contre pales empêche la formation d'un vortex. Un gaz est mis en contact avec la solution à l'aide d'un tube plongeant pendant 7 jours à pression atmosphérique. Les essais sont conduits soit en absence soit en présence d'un agent inhibiteur de dégradation incorporé dans la solution aqueuse d'amine à 0,25% poids.

L'essai est conduit en présence de $CO_2$ et d'oxygène : le gaz mis en contact avec la solution est un mélange de 7Nl/h d'air atmosphérique, c'est-à-dire de l'air ambiant non purifié, et de 0,033 Nl/h de $CO_2$ réalisé dans une chambre de mélange. Le gaz contient donc du $CO_2$, de l'azote et de l'oxygène, la teneur en oxygène dans le gaz étant de 21% environ.

Une analyse par chromatographie en phase gazeuse de la solution ainsi dégradée est réalisée à la fin de l'essai. La méthode chromatographique utilise une colonne polaire, un gaz vecteur, l'hélium, un étalon interne, le triéthylèneglycol et une détection FID (Flame Induced Detection). Cette analyse permet de déterminer la concentration résiduelle d'amine et donc le taux dégradation défini par :

$$taux_de_dégradation = \left(1 - \frac{[amine]finale}{[amine]initiale}\right) * 100$$

Le tableau 4 donne les taux de dégradation de la DEA et de l'AMP en présence d'agents inhibiteurs de dégradation ou non :

- Cas n°14 : DEA 40% poids sans additif
- Cas n°15 : DEA 40% poids en présence d'un additif selon l'invention, le 4-méthyl-4H-1,2,4-triazole-3-thiol
- Cas n°16 : AMP 40% poids sans additif
- Cas n°17 : AMP 40% poids en présence d'un additif selon l'invention, le 4-méthyl-4H-1,2,4-triazote-3-thiol

Tableau 4 : comparaison des taux de dégradation de la DEA 40% poids et de l'AMP 40% poids obtenus dans l'eau à 80°C en absence et en présence d'un agent inhibiteur de dégradation selon l'invention.

| CAS | Teneur en $O_2$ | AMINE | Nom de l'additif | Taux de dégradat ion |
|---|---|---|---|---|
| 14 | 21% | DEA | - | 44% |
| 15 | 21% | DEA | 4-méthyl-4H-1,2,4-triazole-3-thiol | <3% |
| 16 | 21% | AMP | - | 8% |
| 17 | 21% | AMP | 4-méthyl-4H-1,2,4-triazole-3-thiol | <3% |

Il apparaît clairement qu'en présence d'un agent inhibiteur de dégradation selon l'invention, la dégradation de la DEA à 40 % poids et de l'AMP à 40% poids dans l'eau peuvent être considérées comme nulles car inférieures à l'incertitude de la mesure qui est de 3%.

En conclusion, les agents inhibiteurs de dégradation selon l'invention combattent efficacement l'effet de l'oxygène sur la dégradation de la DEA et de l'AMP même à forte concentration en amine.

EXEMPLE 6 :

En particulier, les amines peuvent être dégradées par l'oxygène engendrant la formation de produits volatils, qui sont entraînés dans les effluents gazeux du procédé. Ainsi, par exemple dans le cas de la DEA et de l'AMP des quantités préoccupantes d'ammoniac sont formées.

La figure 4 présente un suivi de la concentration en ammoniac dans le gaz sortant du réacteur dans les cas 14 et 15 définis dans l'exemple 5.

La figure 5 présente un suivi de la concentration en ammoniac dans le gaz sortant du réacteur dans les cas 16 et 17 définis dans l'exemple 5.

[A] correspond à la concentration en ammoniac en ppm volume dans le gaz de sortie du réacteur, t représente le temps exprimé en jours. Les cas 14, 15, 16 et 17 sont représentés respectivement par la courbe 14 avec des triangles noirs, la courbe 15 avec des triangles blancs,, la courbe 16 avec des losanges noirs et la courbe 17 avec des losanges blancs.

La concentration en ammoniac dans le gaz sortant du réacteur est déterminée par une analyse en ligne par spectrométrie Infra-Rouge à Transformée de Fourrier.

Dans le cas des agents inhibiteurs de dégradation selon l'invention, la teneur en $NH_3$ est toujours inférieure à 10ppm pendant toute la durée du test (voir courbes 15 et 17) alors que sans agent inhibiteur de dégradation elle atteint près de 300ppm dans le cas 14 et près de 110 ppm dans le cas 16.

Cet exemple montre donc bien que les agents inhibiteurs de dégradation selon l'invention sont efficaces pour réduire les émissions de composés de dégradation volatils des amines, en particulier dans le cas de la DEA 40% poids dans l'eau et dans le cas de l'AMP 40% poids dans l'eau. Par conséquent, dans un procédé industriel utilisant une solution absorbante contenant des agents inhibiteurs de dégradation selon l'invention, les émissions de composés volatils seront bien moindres qu'en absence d'agents inhibiteurs de dégradation.

## Revendications

**1.** Solution absorbante pour absorber les composés acides d'un effluent gazeux, ladite solution comportant :

a) au moins une amine,
b) de l'eau,
c) au moins un composé inhibiteur de dégradation pour limiter la dégradation de ladite amine, le composé inhibiteur de dégradation étant un dérivé d'un triazole ou d'un tétrazole, dont au moins un substituant comporte un atome de soufre.

**2.** Solution absorbante selon la revendication 1, dans laquelle ledit composé inhibiteur de dégradation répond à l'une des formules générales suivantes :

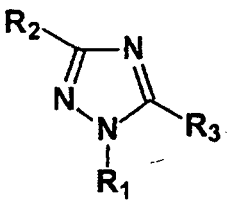 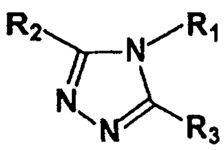 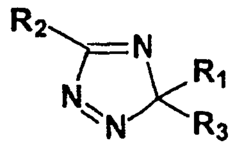

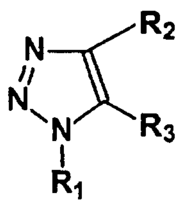 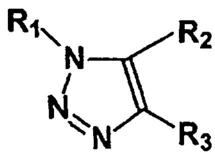 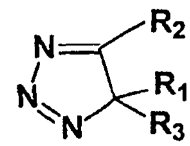

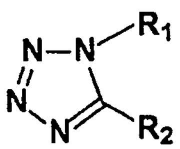 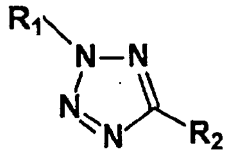 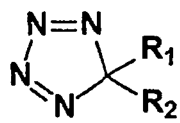

dans laquelle le radical $R_1$ est choisi parmi :

- ○ un atome d'hydrogène,
- ○ un radical hydrocarboné comprenant 1 à 12 atomes de carbone,
- ○ un radical amino de formule générale $-NR_4R_5$ dans lequel le radical $R_4$ et le radical $R_5$ sont indépendamment choisis parmi :
    - ○ un atome d'hydrogène,
    - ○ un radical hydrocarboné comprenant 1 à 12 atomes de carbone,

et dans laquelle chacun des radicaux $R_2$ et $R_3$ est choisi indépendamment parmi les éléments suivants :

a) -S-X dans lequel le radical X est choisi parmi :

- ○ un atome d'hydrogène,
- ○ un élément alcalin ou alcalino-terreux,
- ○ un métal monovalent ou multivalent,
- ○ un cation ammonium NH4+ ou résultant de la protonation d'une fonction amine,
- ○ un cation phosphonium,
- ○ un radical hydrocarboné comprenant 1 à 12 atomes de carbone,
- ○ un radical choisi parmi un radical thiyl-triazole, thio-triazole, thiyl-tétrazole et thio-tétrazole,

b) un atome d'hydrogène,
c) un radical hydroxyle,
d) un radical amino de formule générale $-NR_4R_5$ dans lequel le radical $R_4$ et le radical $R_5$ sont indépendamment choisis parmi :

- ○ un atome d'hydrogène,
- ○ un radical hydrocarboné comprenant 1 à 12 atomes de carbone,

e) un radical comprenant 1 à 12 atomes de carbone.

**3.** Solution absorbante selon la revendication 2, dans laquelle au moins l'un des radicaux $R_1$, $R_2$, $R_3$, $R_4$, $R_5$ et X est un groupe hydrocarboné renfermant entre 1 et 12 atomes de carbone et contient, en outre, au moins un composé choisi parmi un hétéroatome et un halogène.

**4.** Solution absorbante selon la revendication 3, dans laquelle au moins l'un des radicaux $R_1$, $R_2$, $R_3$, $R_4$, $R_5$ et X comporte au moins une fonction choisie parmi le groupe : une fonction hydroxyle, une fonction cétone, une fonction carboxylique, une fonction amine et une fonction nitrile.

**5.** Solution absorbante selon l'une des revendications 2 à 4, dans laquelle au moins deux radicaux choisis parmi $R_1$, $R_2$, $R_3$, $R_4$, $R_5$ et X sont des groupements hydrocarbonés reliés par une liaison covalente pour former un cycle constitué de 5, 6, 7 ou 8 atomes.

**6.** Solution absorbante selon l'une des revendications précédente, dans laquelle la solution comporte entre 10% et 99% poids d'amine, entre 1% et 90% poids d'eau et entre 5 ppm et 5% poids de composé inhibiteur de dégradation.

**7.** Solution absorbante selon l'une des revendications précédentes, dans laquelle le composé inhibiteur de dégradation est choisi parmi le groupe contenant : le 1H-1,2,4-triazole-3-thiol, un sel du 1H-1,2,4-triazole-3-thiol, le 5-phényl-1H-1,2,4-triazole-3-thiol, un sel du 5-phényl-1H-1,2,4-triazole-3-thiol, le 5-(4-pyridyl)-1H-1,2,4-triazole-3-thiol, un sel du 5-(4-pyridyl)-1H-1,2,4-triazole-3-thiol, le 5-(3-pyridyl)-1H-1,2,4-triazole-3-thiol, un sel du 5-(3-pyridyl)-1H-1,2,4-triazole-3-thiol, le 4-méthyl-4H-1,2,4-triazole-3-thiol, un sel du 4-méthyl-4H-1,2,4-triazole-3-thiol, le 4-méthyl-5-(2-thienyl)-4H-1,2,4-triazole-3-thiol, un sel du 4-méthyl-5-(2-thienyl)-4H-1,2,4-triazole-3-thiol, le 4-méthyl-5-(3-thienylméthyl)-4H-1,2,4-triazole-3-thiol, un sel du 4-méthyl-5-(3-thienylméthyl)-4H-1,2,4-triazole-3-thiol, le 4-cyclohexyl-5-sulfanyl-4H-1,2,4-triazol-3-ol, un sel du 4-cyclohexyl-5-sulfanyl-4H-1,2,4-triazol-3-ol, le 3-amino-1,2,4-triazole-5-thlol, un sel du 3-amino-1,2,4-triazole-5-thiol, le 4-amino-4H-1,2,4-triazole-3,5-dithiol, un sel du 4-amino-4H-1,2,4-triazole-3,5-dithiol, le [1,2,4]triazolo[4,3-a]pyridine-3-thiol, un sel du [1,2,4]triazolo[4,3-a]pyridine-3-thiol, le 1H-5-mercapto-1,2,3-triazole, un sel du 1H-5-mercapto-1,2,3-triazole, le 1-méthyl-1H-tétrazole-5-thiol, un sel du 1-méthyl-1H-tétrazole-5-thiol, le 1-éthyl-1H-tétrazole-5-thiol, un sel du 1-éthyl-1H-tétrazole-5-thiol, le 1-phényl-1H-tétrazole-5-thiol, un sel du 1-phényl-1H-tétrazole-5-thiol, le 1-(4-hydroxyphényl)-1H-tétrazole-5-thiol, un sel du 1-(4-hydroxyphényl)-1H-tétrazole-5-thiol, l'acide 5-mercapto-1-tétrazoleacétique, un sel de l'acide 5-mercapto-1-tétrazoleacétique, le 1-[2-(diméthylamino)éthyl]-1H-tétrazole-5-thiol, un sel du 1-[2-(diméthylamino)éthyl]-1H-tétrazole-5-thiol, le 3-Amino-5-methylthio-1H-1,2,4-triazole, un sel du 3-Amino-5-methylthio-1H-1,2,4-triazole, le 5-(méthylthio)-1 H-tétrazole, un sel du 5-(méthylthio)-1H-tétrazole, le 5-(éthylthio)-1H-tétrazole, un sel du 5-(éthylthio)-1H-tétrazole, le 1-méthyl-5-(méthylthio)-1H-tétrazole, un sel du 1-méthyl-5-(méthylthio)-1H-tétrazole, le 4-phényl-4H-1,2,4-triazole-3-thiol, un sel du 4-phényl-4H-1,2,4-triazole-3-thiol, le 5-méthyl-4H-1,2,4-triazole-3-thiol, un sel du 5-méthyl-4H-1,2,4-triazole-3-thiol, la 5-(trifluorométhyl)-2,4-dihydro-3H-1,2,4-triazole-3-thione, un sel de la 5-(trifluorométhyl)-2,4-dihydro-3H-1,2,4-triazole-3-thione, le 4-isopropyl-4H-1,2,4-triazole-3-thiol et un sel du 4-isopropyl-4H-1,2,4-triazole-3-thiol.

**8.** Solution absorbante selon l'une des revendications précédentes, dans laquelle l'amine est choisie parmi le groupe contenant : la N,N,N',N',N"-pentaméthyldiéthylènetriamine, la pipérazine, la monoéthanolamine, la diéthanolamine, la méthyldiéthanolamine, la diisopropanolamine, la diglycolamine, le 2-amino-2-méthylpropanol-1, un sel de la glycine et un sel de la taurine.

**9.** Solution absorbante selon l'une des revendications précédentes, dans laquelle l'amine est choisie parmi la monoéthanolamine, la diéthanolamine et le 2-amino-2-méthylpropanol-1 et dans laquelle le composé inhibiteur de dégradation est choisi parmi le 4-méthyl-4H-1,2,4-triazole-3-thiol, le 1 H-1,2,4-triazole-3-thiol, le 1H-5-mercapto-1,2,3-triazole, le 5-mercapto-1-méthyltétrazole, le 5-méthyl-4H-1,2,4-triazole-3-thiol, le 4-isopropyl-4H-1,2,4-triazole-3-thiol, le 4-phényl-4H-1,2,4-triazole-3-thiol, le 1-[2-(diméthylamino)éthyl]-1H-tétrazole-5-thiol, le sel de sodium du 4-méthyl-4H-1,2,4-triazole-3-thiol, le sel de sodium du 1H-1,2,4-triazole-3-thiol, le sel de sodium du 1H-5-mercapto-1,2,3-triazole, le sel de sodium du 5-mercapto-1-méthyltétrazole, le sel de sodium du 5-méthyl-4H-1,2,4-triazole-3-thiol, le sel de sodium du 4-isopropyl-4H-1,2,4-triazole-3-thiol, le sel de sodium du 4-phényl-4H-1,2,4-triazole-3-thiol, le sel de potassium du 5-méthyl-4H-1,2,4-triazole-3-thiol, le sel de potassium du 4-méthyl-4H-1,2,4-triazole-3-thiol, le sel de potassium du 1 H-1,2,4-triazole-3-thiol, le sel de potassium du 4-isopropyl-4H-1,2,4-triazole-3-thiol, le sel de potassium du 4-phényl-4H-1,2,4-triazole-3-thiol et le sel de potassium du 5-mercapto-1-méthyltétrazole.

**10.** Solution absorbante selon l'une des revendications 8 et 9, comportant au moins 39% poids de monoéthanolamine.

**11.** Procédé pour absorber des composés acides contenus dans un effluent gazeux, dans lequel on met en contact l'effluent gazeux avec une solution aqueuse comportant au moins une amine, et dans lequel on contrôle la dégradation de ladite amine en introduisant au moins un composé inhibiteur de dégradation dérivé d'un triazole ou d'un tétrazole, comportant au moins un substituant comportant un atome de soufre.

**12.** Procédé selon la revendication 11, dans lequel la solution aqueuse est mise en oeuvre pour absorber des composés acides contenus dans l'un des effluents du groupe contenant le gaz naturel, les fumées de combustion, les gaz de synthèse, les gaz de raffinerie, les gaz obtenus en queue du procédé Claus, les gaz de fermentation de biomasse, les gaz de cimenterie et les fumées d'incinérateur.

**13.** Procédé selon la revendication 12, dans lequel l'effluent gazeux comporte au moins 500ppm volumique d'oxygène.

**14.** Procédé selon l'une des revendications 11 à 13, dans lequel on ajoute dans la solution aqueuse au moins un composé inhibiteur de dégradation choisi parmi le groupe contenant: le 1H-1,2,4-triazole-3-thiol, un sel du 1 H-1,2,4-triazole-3-thiol, le 5-phényl-1H-1,2,4-triazole-3-thiol, un sel du 5-phényl-1H-1,2,4-triazole-3-thiol, le 5-(4-pyridyl)-1H-1,2,4-triazole-3-thiol, un sel du 5-(4-pyridyl)-1H-1,2,4-triazole-3-thiol, le 5-(3-pyridyl)-1H-1,2,4-triazole-3-thiol, un sel du 5-(3-pyridyl)-1H-1,2,4-triazole-3-thiol, le 4-méthyl-4H-1,2,4-triazole-3-thiol, un sel du 4-méthyl-4H-1,2,4-triazole-3-thiol, le 4-méthyl-5-(2-thienyl)-4H-1,2,4-triazole-3-thiol, un sel du 4-méthyl-5-(2-thienyl)-4H-1,2,4-triazole-3-thiol, le 4-méthyl-5-(3-thienylméthyl)-4H-1,2,4-triazole-3-thiol, un sel du 4-méthyl-5-(3-thienylméthyl)-4H-1,2,4-triazole-3-thiol, le 4-cyclohexyl-5-sulfanyl-4H-1,2,4-triazol-3-ol, un sel du 4-cyclohexyl-5-sulfanyl-4H-1,2,4-triazol-3-ol, le 3-amino-1,2,4-triazole-5-thiol, un sel du 3-amino-1,2,4-triazole-5-thiol, le 4-amino-4H-1,2,4-triazole-3,5-dithiol, un sel du 4-amino-4H-1,2,4-triazole-3,5-dithiol, le [1,2,4]triazolo[4,3-a]pyridine-3-thiol, un sel du [1,2,4]triazolo[4,3-a]pyridine-3-thiol, le 1H-5-mercapto-1,2,3-triazole, un sel du 1 H-5-mercapto-1,2,3-triazole, le 1-méthyl-1 H-tétrazole-5-thiol, un sel du 1-méthyl-1H-tétrazole-5-thiol, le 1-éthyl-1H-tétrazole-5-thiol, un sel du 1-éthyl-1 H-tétrazole-5-thiol, le 1-phényl-1H-tétrazole-5-thiol, un sel du 1-phényl-1H-tétrazole-5-thiol, le 1-(4-hydroxyphényl)-1H-tétrazole-5-thiol, un sel du 1-(4-hydroxyphényl)-1H-tétrazole-5-thiol, l'acide 5-mercapto-1-tétrazoleacétique, un sel de l'acide 5-mercapto-1-tétrazoleacétique, le 1-[2-(diméthylamino)éthyl]-1H-tétrazole-5-thiol, un sel du 1-[2-(diméthylamino)éthyl]-1H-tétrazole-5-thiol, le 3-Amino-5-methylthio-1 H-1,2,4-triazole, un sel du 3-Amino-5-methylthio-1H-1,2,4-triazole, le 5-(méthylthio)-1H-tétrazole, un sel du 5-(méthylthio)-1H-tétrazole, le 5-(éthylthio)-1H-tétrazole, un sel du 5-(éthylthio)-1H-tétrazole, le 1-méthyl-5-(méthylthio)-1H-tétrazole, un sel du 1-méthyl-5-(méthylthio)-1H-tétrazole, le 4-phényl-4H-1,2,4-triazole-3-thiol, un sel du 4-phényl-4H-1,2,4-triazole-3-thiol, le 5-méthyl-4H-1,2,4-triazole-3-thiol, un sel du 5-méthyl-4H-1,2,4-triazole-3-thiol, la 5-(trifluorométhyl)-2,4-dihydro-3H-1,2,4-triazole-3-thione, un sel de la 5-(trifluorométhyl)-2,4-dihydro-3H-1,2,4-triazole-3-thione, le 4-isopropyl-4H-1,2,4-triazole-3-thiol et un sel du 4-isopropyl-4H-1,2,4-triazole-3-thiol.

**15.** Procédé selon la revendication 11, dans lequel pour limiter la dégradation de l'une des amines choisie parmi la monoéthanolamine, la diéthanolamine et le 2-amino-2-méthylpropanol-1, l'amine étant en solution aqueuse mise en oeuvre pour capter le $CO_2$ des fumées de combustion, on ajoute dans la solution aqueuse au moins un composé inhibiteur de dégradation choisi parmi le groupe contenant: le 4-méthyl-4H-1,2,4-triazole-3-thiol, le 1 H-1,2,4-triazole-3-thiol, le 1H-5-mercapto-1,2,3-triazole, le 5-mercapto-1-méthyltétrazole, le 5-méthyl-4H-1,2,4-triazole-3-thiol, le 4-isopropyl-4H-1,2,4-triazole-3-thiol, le 4-phényl-4H-1,2,4-triazole-3-thiol, le 1-[2-(diméthylamino)éthyl]-1H-tétrazole-5-thiol, le sel de sodium du 4-méthyl-4H-1,2,4-triazole-3-thiol, le sel de sodium du 1 H-1,2,4-triazole-3-thiol, le sel de sodium du 1H-5-mercapto-1,2,3-triazole, le sel de sodium du 5-mercapto-1-méthyltétrazole, le sel de sodium du 5-méthyl-4H-1,2,4-triazole-3-thiol, le sel de sodium du 4-isopropyl-4H-1,2,4-triazole-3-thiol, le sel de sodium du 4-phényl-4H-1,2,4-triazole-3-thiol, le sel de potassium du 5-méthyl-4H-1,2,4-triazole-3-thiol, le sel de potassium du 4-méthyl-4H-1,2,4-triazole-3-thiol, le sel de potassium du 1H-1,2,4-triazole-3-thiol, le sel de potassium du 4-isopropyl-4H-1,2,4-triazole-3-thiol, le sel de potassium du 4-phényl-4H-1,2,4-triazole-3-thiol et le sel de potassium du 5-mercapto-1-méthyltétrazole.

**Patentansprüche**

**1.** Absorptionslösung zur Absorption von sauren Verbindungen eines gasförmigen Abflusses, wobei die Lösung Folgendes umfasst:

a) mindestens ein Amin,
b) Wasser,
c) mindestens eine den Abbau hemmende Verbindung, um den Abbau des Amins zu begrenzen, wobei die den Abbau hemmende Verbindung ein Triazol- oder Tetrazolderivat ist, wobei mindestens ein Substituent ein Schwefelatom umfasst.

**2.** Absorptionslösung nach Anspruch 1, wobei die den Abbau hemmende Verbindung einer der folgenden allgemeinen Formeln entspricht:

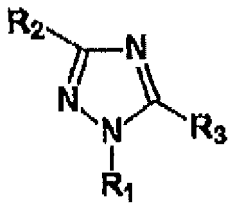 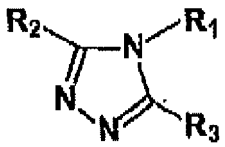 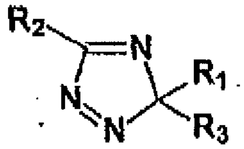

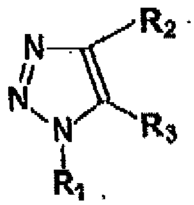 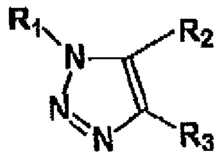 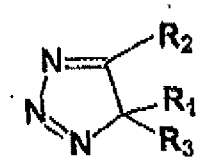

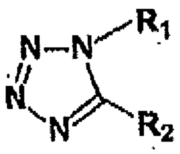 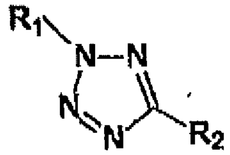 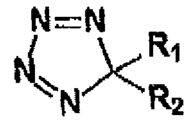

wobei die Gruppe $R_1$ ausgewählt ist aus:

○ einem Wasserstoffatom,
○ einer Kohlenwasserstoffgruppe, umfassend 1 bis 12 Kohlenstoffatome,
o einer Aminogruppe mit der allgemeinen Formel $-NR_4R_5$, wobei die Gruppe $R_4$ und die Gruppe $R_5$ unabhängig ausgewählt sind aus:

o einem Wasserstoffatom,
o einer Kohlenwasserstoffgruppe, umfassend 1 bis 12 Kohlenstoffatome,

und wobei jede der Gruppen $R_2$ und $R_3$ unabhängig aus den folgenden Elementen ausgewählt ist:

a) -S-X, wobei die Gruppe X ausgewählt ist aus:

o einem Wasserstoffatom,
o einem alkalischen oder erdalkalischen Element,
o einem ein- oder mehrwertigen Metall,
o einem Ammoniumkation NH4+ oder resultierend aus der Protonierung einer Aminfunktion,
o einem Phosphoniumkation,
o einer Kohlenwasserstoffgruppe, umfassend 1 bis 12 Kohlenstoffatome,
o einer Gruppe, ausgewählt aus einer Thiyltriazol-, Thiotriazol-, Thiyltetrazol- und Thiotetrazolgruppe,

b) einem Wasserstoffatom,
c) einem Hydroxylradikal,
d) einer Aminogruppe mit der allgemeinen Formel $-NR_4R_5$, wobei die Gruppe $R_4$ und die Gruppe $R_5$ unabhängig ausgewählt sind aus:

o einem Wasserstoffatom,
o einer Kohlenwasserstoffgruppe, umfassend 1 bis 12 Kohlenstoffatome,

e) einer Gruppe, umfassend 1 bis 12 Kohlenstoffatome.

**3.** Absorptionslösung nach Anspruch 2, wobei mindestens einer der Reste $R_1$, $R_2$, $R_3$, $R_4$, $R_5$ und X eine Kohlenwasserstoffgruppe ist, die zwischen 1 und 12 Kohlenstoffatomen umfasst und ferner mindestens eine Verbindung enthält,

ausgewählt aus einem Heteroatom und einem Halogen.

4. Absorptionslösung nach Anspruch 3, wobei mindestens eine Gruppe $R_1$, $R_2$, $R_3$, $R_4$, $R_5$ und X mindestens eine Funktion umfasst, ausgewählt aus der Gruppe: eine Hydroxylfunktion, eine Ketonfunktian, eine Garboxylfunktion, eine Aminfunktion und eine Nitrilfunktion.

5. Absorptionslösung nach einem der Ansprüche 2 bis 4, wobei mindestens zwei Gruppen, ausgewählt aus $R_1$, $R_2$, $R_3$, $R_4$, $R_5$ und X, Kohlenwasserstoffgruppen sind, die über eine kovalente Bindung verbunden sind, um eine zyklische Verbindung zu bilden, die aus 5, 6, 7 oder 8 Atomen besteht.

6. Absorptionslösung nach einem der vorhergehenden Ansprüche, wobei die Lösung zwischen 10 Gew.-% und 99 Gew.-% Amin, zwischen 1 Gew.-% und 90 Gew.-% Wasser und zwischen 5 Gew.-ppm und 5 Gew.-% der den Abbau hemmenden Verbindung umfasst.

7. Absorptionslösung nach einem der vorhergehenden Ansprüche, wobei die den Abbau hemmende Verbindung ausgewählt ist aus der Gruppe, enthaltend: 1H-1,2,4-Triazol-3-thiol, ein Salz von 1H-1,2,4-Triazol-3-thiol, 5-Phenyl-1H-1,2,4-triazol-3-thiol, ein Salz von 5-Phenyl-1H-1,2,4-triazol-3-thiol, 5-(4-Pyridyl)-1H-1,2,4-triazol-3-thiol, ein Salz von 5-(4-Pyridyl)-1H-1,2,4-triazol-3-thiol, 5-(3-Pyridyl)-1H-1,2,4-triazol-3-thiol, ein Salz von 5-(3-pyridyl)-1H-1,2,4-triazol-3-thiol, 4-Methyl-4H-1,2,4-triazol-3-thiol, ein Salz von 4-Methyl-4H-1,2,4-triazol-3-thiol, 4-Methyl-5-(2-thienyl)-4H-1,2,4-triazol-3-thiol, ein Salz von 4-Methyl-5-(2-thienyl)-4H-1,2,4-triazol-3-thiol, 4-Methyl-5-(3-thienylmethyl)-4H-1,2,4-triazol-3-thiol, ein Salz von 4-Methyl-6-(3-thienylmethyl)-4H-1,2,4-triazol-3-thiol, 4-Cyclohexyl-5-sulfonyl-4H-1,2,4-triazol-3-ol, ein Salz von 4-Cyclohexyl-5-sulfanyl-4H-1,2,4-triazol-3-ol, 3-Amino-1,2,4-triazol-5-thiol, ein Salz von 3-Amino-1,2,4-triazol-5-thiol, 4-Amino-4H-1,2,4-triazol-3,5-dithiol, ein Salz von 4-Amino-4H-1,2,4-triazol-3,5-dithiol, [1,2,4]Triazolo[4,3-a]pyridin-3-thiol, ein Salz von [1,2,4]Triazolo[4,3-a]pyridin-3-thiol, 1H-5-Mercapto-1,2,3-triazol, ein Salz von 1H-5-Mercapto-1,2,3-triazol, 1-Methyl-1H-tetrazol-6-thiol, ein Salz von 1-Methyl-1H-tetrazol-5-thiol, 1-Ethyl-1H-tetrazol-5-thiol, ein Salz von 1-Ethyl-1H-tetrazol-5-thiol, 1-Phenyl-1H-tetrazol-5-thiol, ein Salz von 1-Phenyl-1H-tetrazol-5-thiol, 1-(4-Hydroxyphonyl)-1H-tetrazol-5-thiol, ein Salz von 1-(4-Hydroxyphenyl)-1H-tetrazo)-5-thiol, 5-Mercapto-1-tetrazolessigssäure, ein Salz von 5-Mercapto-1-tetrazolessigssäure, 1-[2-(Dimethylamino) ethyl]-1H-tetrazol-5-thiol, ein Salz von 1-[2-(Dimethylamino)ethyl]-1H-tetrazol-5-thiol, 3-Amino-5-methylthio-1H-1,2,4-triazol, ein Salz von 3-Amino-5-methylthio-1H-1,2,4-triazol, 5-(Methylthio)-1H-tetrazol, ein Salz von 5-(Methylthio)-1H-tetrazol, 5-(Ethylthio)-1 H-tetrazol, ein Salz von 5-(Ethylthio)-1 H-tetrazol, 1-Methyl-5-(methylthio)-1H-tetrazol, ein Salz von 1-Methyl-5-(methylthio)-1H-tetrazol, 4-Phenyl-4H-1,2,4-triazol-3-thiol, ein Salz von 4-Phenyl-4H-1,2,4-triazol-3-thiol, 5-Methyl-4H-1,2,4-triazol-3-thiol, ein Salz von 5-Methyl-4H-1,2,4-triazol-3-thiol, 5-(Trifluormethyl)-2,4-dihydro-3H-1,2,4-triazol-3-thion, ein Salz von 5-(Trifluormethyl)-2,4-dihydro-3H-1,2,4-triazol-3-thion, 4-Isopropyl-4H-1,2,4-triazol-3-thiol und ein Salz von 4-Isopropyl-4H-1,2,4-triazol-3-thiol.

8. Absorptionslösung nach einem der vorhergehenden Ansprüche, wobei das Amin ausgewählt ist aus der Gruppe, enthaltend: N,N,N',N',N"-Pentamethyldiethylentriamin, Piperazin, Monoethanolamin, Diethanolamin, Methyldiethanolamin, Diisopropanolamin, Diglycolamin, 2-Amino-2-methylpropanol-1, ein Glycinsalz und ein Taurinsalz.

9. Absorptionslösung nach einem der vorhergehenden Ansprüche, wobei das Amin ausgewählt ist aus Monoethanolamin, Diethanolamin und 2-Amino-2-methylpropanol-1 und wobei die den Abbau hemmende Verbindung ausgewählt ist aus 4-Methyl-4H-1,2,4-triazol-3-thiol, 1H-1,2,4-Triazol-3-thiol, 1 H-5-Mercapto-1,2,3-triazol, 5-Mercapto-1-methyltetrazol, 5-Methyl-4H-1,2,4-triazol-3-thiol, 4-Isopropyl-4H-1,2,4-triazol-3-thiol, 4-Phenyl-4H-1,2,4-triazol-3-thiol, 1-[2-(Dimethylamino)ethyl]-1H-tetrazol-5-thiol, dem Natriumsalz von 4-Methyl-4H-1,2,4-triazol-3-thiol, dem Natriumsalz von 1H-1,2,4-Triazol-3-thiol, dem Natriumsalz von 1H-5-Mercapto-1,2,3-triazol, dem Natriumsalz von 5-Mercapto-1-methyltetrazol, dem Natriumsalz von 5-Methyl-4H-1,2,4-triazol-3-thiol, dem Natriumsalz von 4-Isopropyl-4H-1,2,4-triazol-3-thiol, dem Natriumsalz von 4-Phenyl-4H-1,2,4-triazol-3-thiol, dem Kaliumsalz von 5-Methyl-4H-1,2,4-triazol-3-thiol, dem Kaliumsalz von 4-Methyl-4H-1,2,4-triazol-3-thiol, dem Kaliumsalz von 1H-1,2,4-Triazol-3-thiol, dem Kaliumsalz von 4-Isopropyl-4H-1,2,4-triazol-3-thiol, dem Kaliumsalz von 4-Phenyl-4H-1,2,4-triazol-3-thiol und dem Kaliumsalz von 5-Mercapto-1-methyltetrazol.

10. Absorptionslösung nach einem der Ansprüche 8 und 9, umfassend mindestens 39 Gew.-% Monoethanolamin.

11. Verfahren zur Absorption von Säureverbindungen, die in einem gasförmigen Abfluss enthalten sind, wobei der gasförmige Abfluss mit einer wässrigen Lösung in Kontakt gebracht wird, die mindestens ein Amin umfasst, und wobei der Abbau des Amins kontrolliert wird, indem mindestens eine den Abbau hemmende Verbindung eingeführt wird, die von einem Triazol oder einem Tetrazol abgeleitet ist, die mindestens einen Substituenten umfasst, der ein

Schwefelatom umfasst.

12. Verfahren nach Anspruch 11, wobei die wässrige Lösung eingesetzt wird, um Säureverbindungen zu absorbieren, die in einem der Abflüsse der Gruppe enthalten sind, enthaltend Erdgas, Verbrennungsrauchgase, Synthesegase, Raffineriegase, Gase, die am Ende des Claus-Verfahrens erhalten werden, Gase aus der Biomassefermentation, Gase aus dem Zementwerk und Rauchgase einer Verbrennungsanlage.

13. Verfahren nach Anspruch 12, wobei der gasförmige Abfluss mindestens 500 Vol.-ppm Sauerstoff umfasst.

14. Verfahren nach einem der Ansprüche 11 bis 13, wobei zu der wässrigen Lösung mindestens eine den Abbau hemmende Verbindung zugegeben wird, ausgewählt aus der Gruppe, enthaltend: 1H-1,2,4-Triazol-3-thiol, ein Salz von 1H-1,2,4-Triazol-3-thiol, 5-Phenyl-1H-1,2,4-triazol-3-thiol, ein Salz von 5-Phenyl-1H-1,2,4-triazol-3-thiol, 5-(4-Pyridyl)-1H-1,2,4-triazol-3-thiol, ein Salz von 6-(4-Pyridyl)-1H-1,2,4-triazol-3-thiol, 5-(3-Pyridyl)-1H-1,2,4-triazol-3-thiol, ein Salz von 5-(3-pyridyl)-1H-1,2,4-triazol-3-thiol, 4-Methyl-4H-1,2,4-triazol-3-thiol, ein Salz von 4-Methyl-4H-1,2,4-triazol-3-thiol, 4-Methyl-5-(2-thienyl)-4H-1,2,4-triazol-3-thiol, ein Salz von 4-Methyl-5-(2-thienyl)-4H-1,2,4-triazol-3-thiol, 4-Methyl-5-(3-thienylmethyl)-4H-1,2,4-triazol-3-thiol, ein Salz von 4-Methyl-5-(3-thienylmethyl)-4H-1,2,4-triazol-3-thiol, 4-Cyclohexyl-5-sulfanyl-4H-1,2,4-triazol-3-ol, ein Salz von 4-Cyclohexyl-5-sulfanyl-4H-1,2,4-triazol-3-ol, 3-Amino-1,2,4-triazol-5-thiol, ein Salz von 3-Amino-1,2,4-triazol-5-thiol, 4-Amino-4H-1,2,4-triazol-3,5-dithiol, ein Salz von 4-Amino-4H-1,2,4-triazol-3,5-dithiol, [1,2,4]Triazolo[4,3-a]pyridin-3-thiol, ein Salz von [1,2,4]Triazolo [4,3-a]pyridin-3-thiol, 1 H-5-Mercapto-1,2,3-triazol, ein Salz von 1H-5-Mercapto-1,2,3-triazol, 1-Methyl-1H-tetrazol-5-thiol, ein Salz von 1-Methyl-1H-tetrazol-5-thiol, 1-Ethyl-1H-tetrazol-5-thiol, ein Salz von 1-Ethyl-1H-tetrazol-5-thiol, 1-Phenyl-1H-tetrazol-5-thiol, ein Salz von 1-Phenyl-1 H-tetrazol-5-thiol, 1-(4-Hydroxyphenyl)-1H-tetrazol-5-thiol, ein Salz von 1-(4-Hydroxyphenyl)-1H-tetrazol-5-thiol, 5-Mercapto-1-tetrazolessigssäure, ein Salz von 5-Mercapto-1-tetrazolessigssäure, 1-[2-(Dimethylamino)ethyl]-1H-tetrazol-5-thiol, ein Salz von 1-[2-(Dimethylamino)ethyl]-1H-tetrazol-5-thiol, 3-Amino-5-methylthio-1H-1,2,4-triazol, ein Salz von 3-Amino-5-methylthio-1H-1,2,4-triazol, 5-(Methylthio)-1H-tetrazol, ein Salz von 5-(Methylthio)-1H-tetrazol, 5-(Ethylthio)-1 H-tetrazol, ein Salz von 5-(Ethylthio)-1H-tetrazol, 1-Methyl-5-(methylthio)-1H-tetrazol, ein Salz von 1-Methyl-5-(methylthio)-1H-tetrazol, 4-Phenyl-4H-1,2,4-triazol-3-thiol, ein Salz von 4-Phenyl-4H-1,2,4-triazol-3-thiol, 5-Methyl-4H-1,2,4-triazol-3-thiol, ein Salz von 5-Methyl-4H-1,2,4-triazol-3-thiol, 5-(Trifluormethyl)-2,4-dihydro-3H-1,2,4-triazol-3-thion, ein Salz von 5-(Trifluormethyl)-2,4-dihydro-3H-1,2,4-triazol-3-thion, 4-Isopropyl-4H-1,2,4-triazol-3-thiol und ein Salz von 4-Isopropyl-4H-1,2,4-triazol-3-thiol.

15. Verfahren nach Anspruch 11, wobei zur Begrenzung des Abbaus eines der Amine, ausgewählt aus Monoethanolamin, Diethanolamin und 2-Amino-2-methylpropanol-1, wobei das Amin in wässriger Lösung gelöst ist, die eingesetzt wird, um das $CO_2$ der Verbrennungsrauchgase zu binden, zu der wässrigen Lösung mindestens eine den Abbau hemmende Verbindung gegeben wird, ausgewählt aus der Gruppe, enthaltend: 4-Methyl-4H-1,2,4-triazol-3-thiol, 1H-1,2,4-Triazol-3-thiol, 1H-5-Mercapto-1,2,3-triazol, 5-Mercapto-1-methyltetrazol, 5-Methyl-4H-1,2,4-triazol-3-thiol, 4-Isopropyl-4H-1,2,4-triazol-3-thiol, 4-Phenyl-4H-1,2,4-triazol-3-thiol, 1-[2-(Dimethylamino)ethyl]-1H-tetrazol-5-thiol, dem Natriumsalz von 4-Methyl-4H-1,2,4-triazol-3-thiol, dem Natriumsalz von 1H-1,2,4-Triazol-3-thiol, dem Natriumsalz von 1H-5-Mercapto-1,2,3-triazol, dem Natriumsalz von 5-Mercapto-1-methyltetrazol, dem Natriumsalz von 5-Methyl-4H-1,2,4-triazol-3-thiol, dem Natriumsalz von 4-Isopropyl-4H-1,2,4-triazol-3-thiol, dem Natriumsalz von 4-Phenyl-4H-1,2,4-triazol-3-thiol, dem Kaliumsalz von 5-Methyl-4H-1,2,4-triazol-3-thiol, dem Kaliumsalz von 4-Methyl-4H-1,2,4-triazol-3-thiol, dem Kaliumsalz von 1H-1,2,4-Triazol-3-thiol, dem Kaliumsalz von 4-Isopropyl-4H-1,2,4-triazol-3-thiol, dem Kaliumsalz von 4-Phenyl-4H-1,2,4-triazol-3-thiol und dem Kaliumsalz von 5-Mercapto-1-methyltetrazol.

**Claims**

1. An absorbent solution for absorbing the acid compounds of a gaseous effluent, said solution comprising:

a) at least one amine,
b) water,
c) at least one degradation inhibiting compound for limiting the degradation of said amine, the degradation inhibiting compound being a derivative of a triazole or of a tetrazole, at least one substituent of which comprises a sulfur atom.

2. An absorbent solution as claimed in claim 1, wherein said degradation inhibiting compound meets one of the following

general formulas:

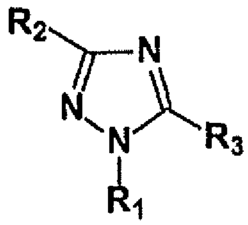
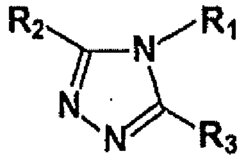
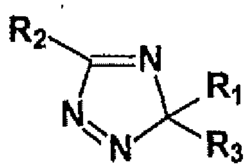
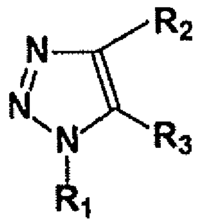
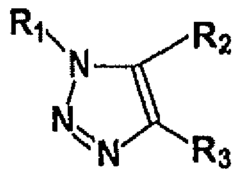
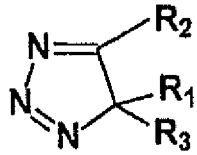
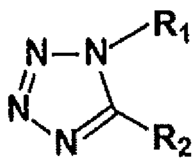
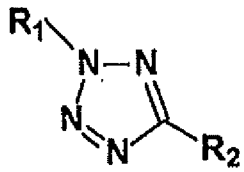
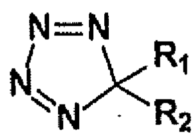

wherein radical $R_1$ is selected from among:

- a hydrogen atom,
- a hydrocarbon radical comprising 1 to 12 carbon atoms,
- an amino radical of general formula $-NR_4R_5$ wherein radical $R_4$ and radical $R_5$ are independently selected from among:
  - a hydrogen atom,
  - a hydrocarbon radical comprising 1 to 12 carbon atoms,

and wherein each radical $R_2$ and $R_3$ is independently selected from among the following elements:

a) -S-X wherein radical X is selected from among:

- a hydrogen atom,
- an alkaline or alkaline-earth element,
- a monovalent or multivalent metal,
- an ammonium cation NH4+ or resulting from the protonation of an amine function,
- a phosphonium cation,
- a hydrocarbon radical comprising 1 to 12 carbon atoms,
- a radical selected from among a thiyl-triazole, thio-triazole, thiyl-tetrazole and thio-tetrazole radical,

b) a hydrogen atom,
c) a hydroxyl radical,
d) an amino radical of general formula $-NR_4R_5$ wherein radical $R_4$ and radical $R_5$ are independently selected from among:

- a hydrogen atom,
- a hydrocarbon radical comprising 1 to 12 carbon atoms,

e) a radical comprising 1 to 12 carbon atoms.

3. An absorbent solution as claimed in claim 2, wherein at least one of radicals $R_1$, $R_2$, $R_3$, $R_4$, $R_5$ and X is a hydrocarbon group containing between 1 and 12 carbon atoms and additionally contains at least one compound selected from among a heteroatom and a halogen.

4. An absorbent solution as claimed in claim 3, wherein at least one of radicals $R_1$, $R_2$, $R_3$, $R_4$, $R_5$ and X comprises at least one function selected from among the group as follows: a hydroxyl function, a ketone function, a carboxylic function, an amine function and a nitrile function.

5. An absorbent solution as claimed in any one of claims 2 to 4, wherein at least two radicals selected from among $R_1$, $R_2$, $R_3$, $R_4$, $R_5$ and X are hydrocarbon groups bonded by a covalent bond so as to form a ring made up of 5, 6, 7 or 8 atoms.

6. An absorbent solution as claimed in any one of the previous claims, wherein the solution comprises between 10 wt. % and 99 wt.% amine, between 1 wit.% and 90 wt.% water, and between 5 ppm and 5 wt.% degradation inhibiting compound.

7. An absorbent solution as claimed in any one of the previous claims, wherein the degradation inhibiting compound is selected from among the group containing: 1H-1,2,4-triazole-3-thiol, a 1H-1,2,4-triazole-3-thiol salt, 5-phenyl-1H-1,2,4-triazole-3-thiol, a 5-phenyl-1H-1,2,4-triazole-3-thiol salt, 5-(4-pyridyl)-1H-1,2,4-triazole-3-thiol, a 5-(4-pyridyl)-1H-1,2,4-triazole-3-thiol salt, 5-(3-pyridyl)-1H-1,2,4-triazole-3-thiol, a 5-(3-pyridyl)-1H-1,2,4-triazole-3-thiol salt, 4-methyl-4H-1,2,4-triazole-3-thiol, a 4-methyl-4H-1,2,4-triazole-3-thiol salt, 4-methyl-5-(2-thienyl)-4H-1,2,4-triazole-3-thiol, a 4-methyl-5-(2-thienyl)-4H-1,2,4-triazole-3-thiol salt, 4-methyl-5-(3-thienylmethyl)-4H-1,2,4-triazole-3-thiol, a 4-methyl-5-(3-thienylmethyl)-4H-1,2,4-triazole-3-thiol salt, 4-cyclohexyl-5-sulfanyl-4H-1,2,4-triazole-3-ol, a 4-cyclohexyl-5-sulfanyl-4H-1,2,4-triazole-3-ol salt, 3-amino-1,2,4-triazole-5-thiol, a 3-amino-1,2,4-triazole-5-thiol salt, 4-amino-4H-1,2,4-triazole-3,5-dithiol, a 4-amino-4H-1,2,4-triazole-3,5-dithiol salt, [1,2,4]triazolo[4,3-a]pyridine-3-thiol, a [1,2,4]triazolo[4,3-a] pyridine-3-thiol salt, 1H-5-mercapto-1,2,3-triazole, a 1H-5-mercapto-1,2,3-triazole salt, 1-methyl-1H-tetrazole-5-thiol, a 1-methyl-1H-tetrazole-5-thiol salt, 1-ethyl-1H-tetrazole-5-thiol, a 1-ethyl-1H-tetrazole-5-thiol salt, 1-phenyl-1H-tetrazole-5-thiol, a 1-phenyl-1H-tetrazole-5-thiol salt, 1-(4-hydroxyphenyl)-1H-tetrazole-5-thiol, a 1-(4-hydroxyphenyl)-1H-tetrazole-5-thiol salt, 5-mercapto-1-tetrazolacetic acid, a 5-mercapto-1-tetrazolacetic acid salt, 1-[2-(dimethylamino)ethyl]-1H-tetrazole-5-thiol, a 1-[2-(dimethylamino)ethyl]-1H-tetrazole-5-thiol salt, 3-amino-5-methylthio-1H-1,2,4-triazole, a 3-amino-5-methylthio-1H-1,2,4-triazole salt, 5-(methylthio)-1H-tetrazole, a 5-(methylthio)-1H-tetrazole salt, 5-(ethylthio)-1H-tetrazole, a 5-(ethylthio)-1H-tetrazole salt, 1-methyl-5-(methylthio)-1H-tetrazole, a 1-methyl-5-(methylthio)-1H-tetrazole salt, 4-phenyl-4H-1,2,4-triazole-3-thiol, a 4-phenyl-4H-1,2,4-triazole-3-thiol salt, 5-methyl-4H-1,2,4-triazole-3-thiol, a 5-methyl-4H-1,2,4-triazole-3-thiol salt, 5-(trifluoromethyl)-2,4-dihydro-3H-1,2,4-triazole-3-thione, a 5-(trifluoromethyl)-2,4-dihydro-3H-1,2,4-triazole-3-thione salt, 4-isopropyl-4H-1,2,4-triazole-3-thiol and a 4-isopropyl-4H-1,2,4-triazole-3-thiol salt.

8. An absorbent solution as claimed in any one of the previous claims, wherein the amine is selected from the group containing: N,N,N',N',N"-pentamethyldiethylene-triamine, piperazine, monoethanolamine, diethanolamine, methyldiethanolamine, diisopropanolamine, diglycolamine, 2-amino-2-methylproponol-1, a glycine salt and a taurine salt.

9. An absorbent solution as claimed in any one of the previous claims, wherein the amine is selected from among monoethanolamine, diethanolamine and 2-amino-2-methylpropanol-1, and wherein the degradation inhibiting compound is selected from among: 4-methyl-4H-1,2,4-triazole-3-thiol, 1H-1,2,4-triazole-3-thiol, 1H-5-mercapto-1,2,3-triazole, 5-mercapto-1-methyltetrazole, 5-methyl-4H-1,2,4-triazole-3-thiol, 4-isopropyl-4H-1,2,4-triazole-3-thiol, 4-phenyl-4H-1,2,4-triazole-3-thiol, 1-[2-(dimethylamino) ethyl]-1H-tetrazole-5-thiol, 4-methyl-4H-1,2,4-triazole-3-thiol sodium salt, 1H-1,2,4-triazole-3-thiol sodium salt, 1H-5-mercapto-1,2,3-triazole sodium salt, 5-mercapto-1-methyltetrazole sodium salt, 5-methyl-4H-1,2,4-triazole-3-thiol sodium salt, 4-isopropyl-4H-1,2,4-triazole-3-thiol sodium salt, 4-phenyl-4H-1,2,4-triazole-3-thiol sodium salt, 5-methyl-4H-1,2,4-triazole-3-thiol potassium salt, 4-methyl-4H-1,2,4-triazole-3-thiol potassium salt, 1H-1,2,4-triazole-3-thiol potassium salt, 4-isopropyl-4H-1,2,4-triazole-3-thiol potassium salt, 4-phenyl-4H-1,2,4-triazole-3-thiol potassium salt and 5-mercapto-1-methyltetrazole potassium salt.

10. An absorbent solution as claimed in any one of claims 8 and 9, comprising at least 39 wt.% monoethanolamine.

11. A method for absorbing acid compounds contained in a gaseous effluent, wherein the gaseous effluent is contacted with an aqueous solution comprising at least one amine, and wherein the degradation of said amine is controlled

by introducing at least one degradation inhibiting compound derived from a triazole or from a tetrazole, comprising at least one substituent containing a sulfur atom.

**12.** A method as claimed in claim 11, wherein the aqueous solution is used for absorbing acid compounds contained in one of the effluents from the group containing natural gas, combustion fumes, syngases, refinery gases, Claus tail gases, biomass fermentation gases, cement plant gases and incinerator fumes.

**13.** A method as claimed in claim 12, wherein the gaseous effluent comprises at least 500 vol.ppm oxygen.

**14.** A method as claimed in any one of claims 11 to 13, wherein at least one degradation inhibiting compound selected from the following group is added to the aqueous solution: 1H-1,2,4-triazole-3-thiol, a 1H-1,2,4-triazole-3-thiol salt, 5-phenyl-1H-1,2,4-triazole-3-thiol, a 5-phenyl-1H-1,2,4-triazole-3-thiol salt, 5-(4-pyridyl)-1H-1,2,4-triazole-3-thiol, a 5-(4-pyridyl)-1H-1,2,4-triazole-3-thiol salt, 5-(3-pyridyl)-1H-1,2,4-triazole-3-thiol, a 5-(3-pyridyl)-1H-1,2,4-triazole-3-thiol salt, 4-methyl-4H-1,2,4-triazole-3-thiol, a 4-methyl-4H-1,2,4-triazole-3-thiol salt, 4-methyl-5-(2-thienyl)-4H-1,2,4-triazole-3-thiol, a 4-methyl-5-(2-thienyl)-4H-1,2,4-triazole-3-thiol salt, 4-methyl-5-(3-thienylmethyl)-4H-1,2,4-triazole-3-thiol, a 4-methyl-5-(3-thienylmethyl)-4H-1,2,4-triazole-3-thiol salt, 4-cyclohexyl-5-sulfanyl-4H-1,2,4-triazole-3-ol, a 4-cyclohexyl-5-sulfanyl-4H-1,2,4-triazole-3-ol salt, 3-amino-1,2,4-triazole-5-thiol, a 3-amino-1,2,4-triazole-5-thiol salt, 4-amino-4H-1,2,4-triazole-3,5-dithiol, a 4-amino-4H-1,2,4-triazole-3,5-dithiol salt, [1,2,4]triazolo [4,3-a]pyridine-3-thiol, a [1,2,4] triazolo[4,3-a]pyridine-3-thiol salt, 1H-5-mercapto-1,2,3-triazole, a 1H-5-mercapto-1,2,3-triazole salt, 1-methyl-1H-tetrazole-5-thiol, a 1-methyl-1H-tetrazole-5-thiol salt, 1-ethyl-1H-tetrazole-5-thiol, a 1-ethyl-1H-tetrazole-5-thiol salt, 1-phenyl-1H-tetrazole-5-thiol, a 1-phenol-1H-tetrazole-5-thiol salt, 1-(4-hydroxyphenyl)-1H-tetrazole-5-thiol, a 1-(4-hydroxyphenyl)-1H-tetrazole-5-thiol salt, 5-mercapto-1-tetrazolacetic acid, a 5-mercapto-1-tetrazolacetic acid salt, 1-[2-(dimethylamino)ethyl]-1H-tetrazole-5-thiol, a 1-[2-(dimethylamino)ethyl)-1H-tetrazole-5-thiol salt, 3-amino-5-methylthio-1H-1,2,4-triazole, a 3-amino-5-methylthio-1H-1,2,4-triazole salt, 5-(methylthio)-1H-tetrazole, a 5-(methylthio)-1H-tetrazole salt, 5-(ethylthio)-1H-tetrazole, a 5-(ethylthio)-1H-tetrazole salt, 1-methyl-5-(methylthio)-1H-tetrazole, a 1-methyl-5-(methylthio)-1H-tetrazole salt, 4-phenyl-4H-1,2,4-triazole-3-thiol, a 4-phenyl-4H-1,2,4-triazole-3-thiol salt, 5-methyl-4H-1,2,4-triazole-3-thiol, a 5-methyl-4H-1,2,4-triazole-3-thiol salt, 5-(trifluoromethyl)-2,4-dihydro-3H-1,2,4-triazole-3-thione, a 5-(trifluoromethyl)-2,4-dihydro-3H-1,2,4-triazole-3-thione salt, 4-isopropyl-4H-1,2,4-triazole-3-thiol and a 4-isopropyl-4H-1,2,4-triazole-3-thiol salt.

**15.** A method as claimed in claim 11 wherein, in order to limit degradation of one of the amines selected from among monoethanolamine, diethanolamine and 2-amino-2-methylpropanol-1, the amine being in aqueous solution used for capturing the $CO_2$ from combustion fumes, at least one degradation inhibiting compound selected from the group containing the following elements is added to the aqueous solution: 4-methyl-4H-1,2,4-triazole-3-thiol, 1H-1,2,4-triazole-3-thiol, 1H-5-mercapto-1,2,3-triazole, 5-mercapto-1-methyl-tetrazole, 5-methyl-4H-1,2,4-triazole-3-thiol, 4-isopropyl-4H-1,2,4-triazole-3-thiol, 4-phenyl-4H-1,2,4-triazole-3-thiol, 1-[2-(dimethylamino)ethyl]-1H-tetrazole-5-thiol, 4-methyl-4H-1,2,4-triazole-3-thiol sodium salt, 1H-1,2,4-triazole-3-thiol sodium salt, 1H-5-mercapto-1,2,3-triazole sodium salt, 5-mercapto-1-methyltetrazole sodium salt, 5-methyl-4H-1,2,4-triazole-3-thiol sodium salt, 4-isopropyl-4H-1,2,4-triazole-3-thiol sodium salt, 4-phenyl-4H-1,2,4-triazole-3-thiol sodium salt, 5-methyl-4H-1,2,4-triazole-3-thiol potassium salt, 4-methyl-4H-1,2,4-triazole-3-thiol potassium salt, 1H-1,2,4-triazole-3-thiol potassium salt, 4-isopropyl-4H-1,2,4-triazole-3-thiol potassium salt, 4-phenyl-4H-1,2,4-triazole-3-thiol potassium salt and 5-mercapto-1-methyltetrazole potassium salt.

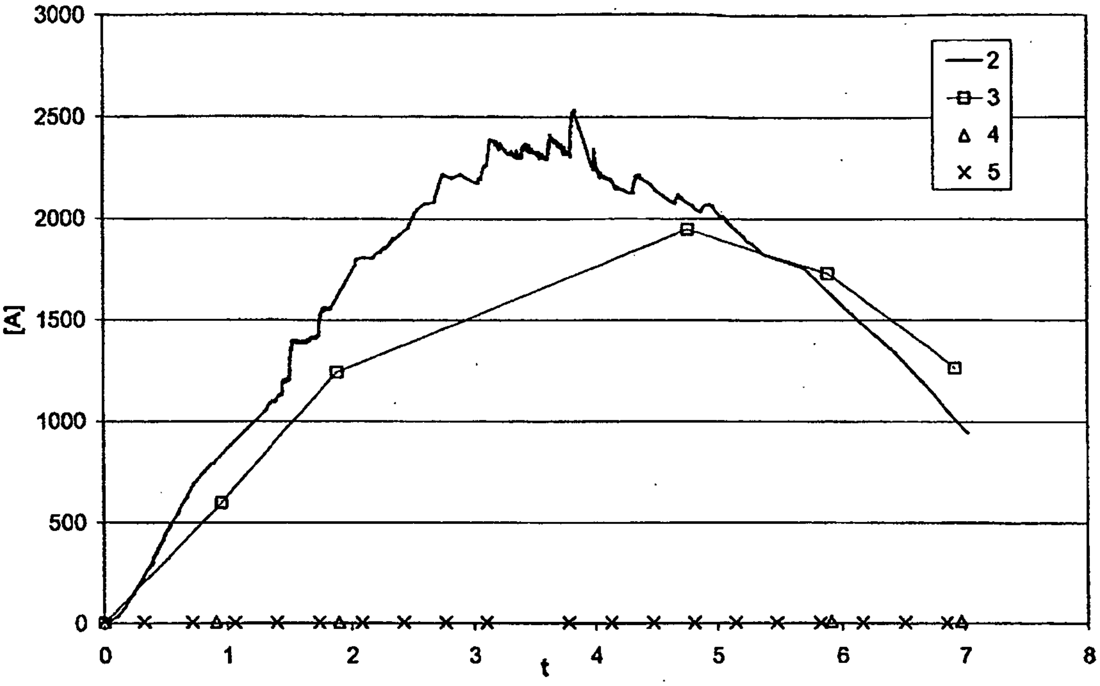
3000
2500
2000
1500
1000
500
0
[A]
0
1
2
3
4
5
6
7
8
t
2
3
4
5

FIG. 1

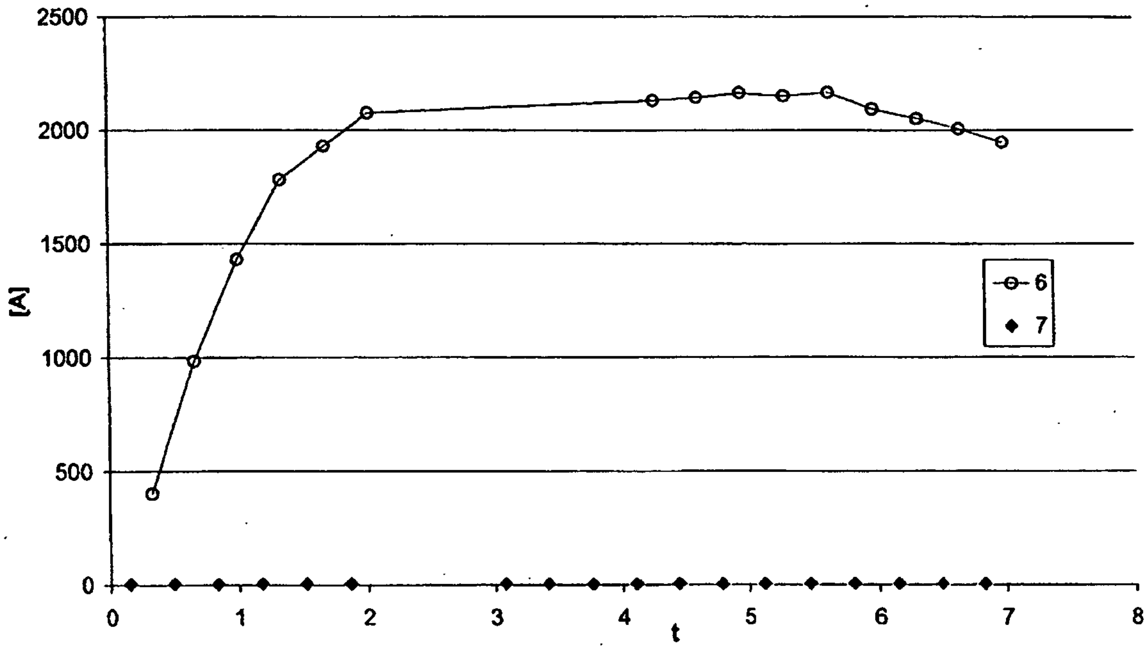
2500
2000
1500
1000
500
0
[A]
0
1
2
3
4
5
6
7
8
t
6
7

FIG. 2

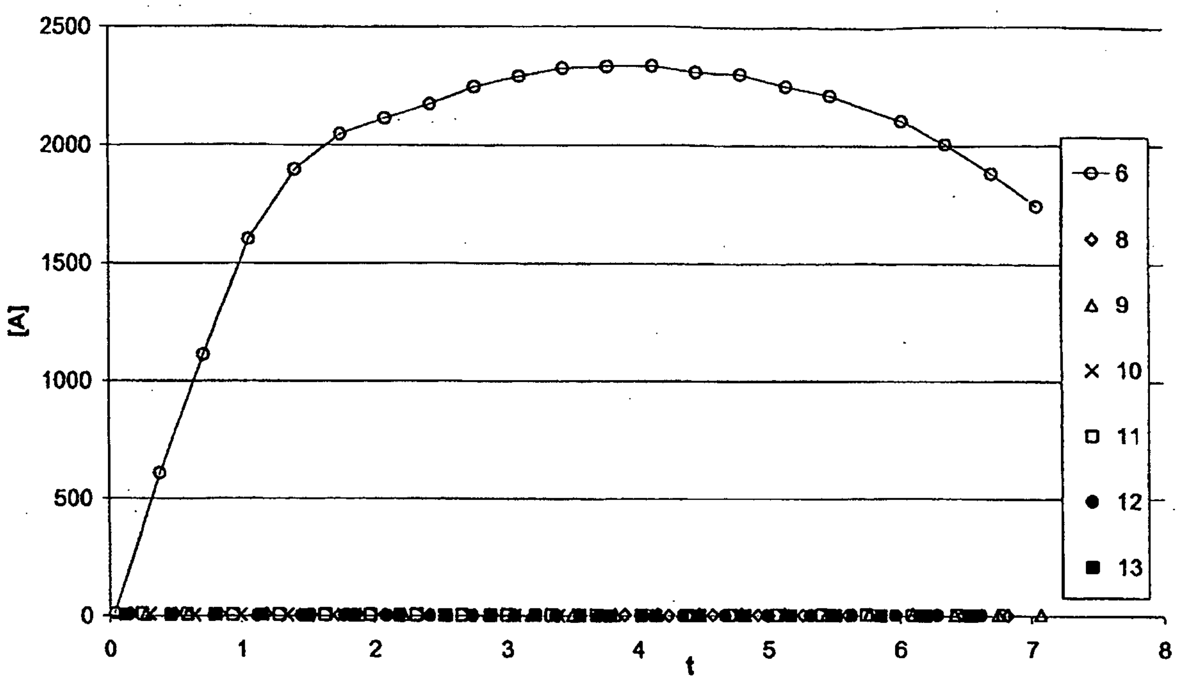
2500
2000
1500
1000
500
0
[A]
0
1
2
3
4
5
6
7
8
t
6
8
9
10
11
12
13

FIG. 3

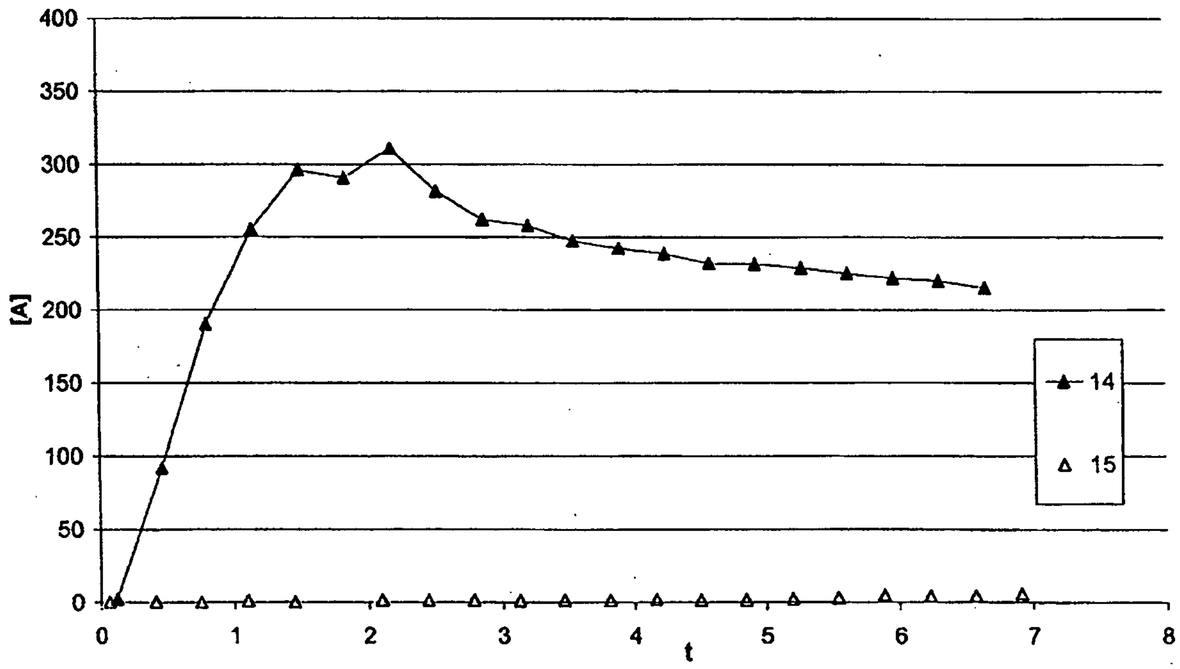
400
350
300
250
200
150
100
50
0
[A]
0
1
2
3
4
5
6
7
8
t
14
15

FIG. 4

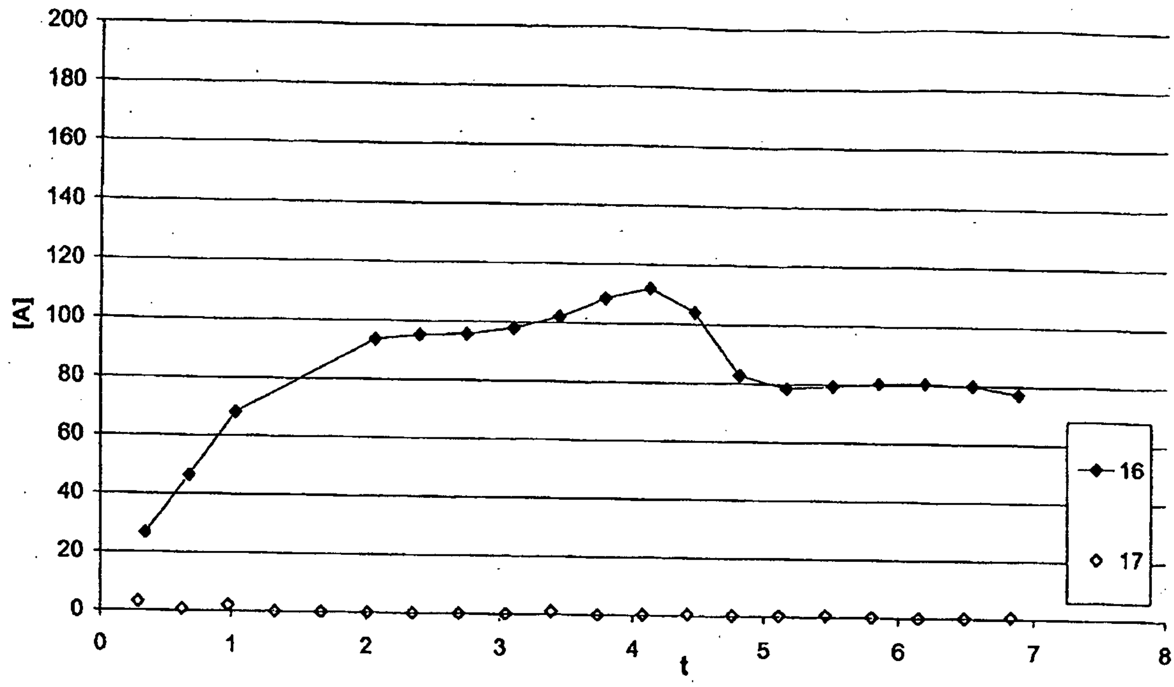


FIG. 5

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- FR 2820430
- US 5686016 A
- US 7056482 B
- EP 1582250 A